# EUROPEAN PATENT APPLICATION

(11) **EP 2 554 048 A1**
(43) Date of publication of application: **06.02.2013**
(21) Application number: 11006347.6
(22) Date of filing: 02.08.2011
(51) Int. Cl.: A01K 67/027

(54) **A genetically modified rodent expressing human CD22**

(71) Applicant: Friedrich-Alexander-Universität Erlangen-Nürnberg, 91054 Erlangen (DE)
(72) Inventor: Nitschke, Lars, 91080 Uttenreuth (DE); Wöhner, Miriam, 91056 Erlangen (DE)
(74) Representative: Vossius & Partner

(57) **Abstract**

The present invention relates to a genetically modified rodent comprising a nucleic acid sequence encoding human CD22, or (a) fragment(s) thereof, in its genome. The present invention further relates to methods of identifying an agent capable of treating diseases associated with dysregulated B lymphocytes as well as a method of testing the safety of an agent for anti-human CD22 therapy, based on the genetically modified rodent of the invention.

## Description

The present invention relates to a genetically modified rodent comprising a nucleic acid sequence encoding human CD22, or (a) fragment(s) thereof, in its genome. The present invention further relates to methods of identifying an agent capable of treating diseases associated with dysregulated B lymphocytes as well as a method of testing the safety of an agent for anti-human CD22 therapy, based on the genetically modified rodent of the invention.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

CD22 is a regulatory molecule that prevents the over-activation of the immune system and the development of autoimmune diseases and functions as an inhibitory co-receptor for BCR-induced Ca²⁺ signalling. CD22 carries inhibitory ITIM signalling motifs, which recruit the tyrosine phosphatase SHP-1 that is responsible for the inhibitory function of CD22. CD22 can bind to α2,6-linked sialic acid-containing ligands in *cis* and in *trans* and the signalling function of CD22 is modulated by this ligand binding (Nitschke, 2009; Walker and Smith, 2008).

CD22 is expressed from the pre B-cell stage to the mature B-cell stage and was shown to be downregulated on plasma cells (Walker and Smith, 2008). Dysregulated B cells have been described to contribute to the pathogenesis of e.g. cancers, such as leukaemia and lymphoma as well as to autoimmune diseases, such as Systemic Lupus Erythematosus (SLE) (Dorner et al., 2009). As CD22 has been shown to be expressed on the majority of all B-cell lymphomas (Pawlak-Byczkowska et al., 1989) and on approx. 65% of the B-cell acute lymphoblastic leukaemia (BLL) (Hurwitz et al., 1988), it has been the target for the development of therapeutics for the treatment of various diseases. For example, CD22 on B cells has been targeted in SLE patients with anti-CD22 antibodies. The humanized monoclonal anti-CD22 antibody Epratuzumab (LymphoCide®) was clinically tested for SLE patients in phase IIb randomized clinical trials. The studies showed a treatment advantage with Epratuzumab over placebo of around 25% (Kalunian et al. 2010). The mode of action of Epratuzumab in SLE treatment is not known, however, mechanisms discussed are for example induction of ADCC, internalisation of CD22 or modulation of B cell signalling (Dorner et al., 2009). All of these mechanisms are derived from testing human peripheral B cells *in vitro.* So far, systematic *in vivo* experiments have not been done, as they could only be done in a limited way in humans. Leukaemia cells have also been targeted by toxins coupled to anti-CD22-antibodies (immunotoxins). As a toxin component, these antibodies are linked to bacterial and plant toxins (Kreitman et al., 2005), RNases (Schirrmann et al., 2009), ribosome inactivating proteins (Flavell et al., 1997) or deglycosylating ricin A chains (Ghetie et al., 1991). Epratuzumab was also successfully tested in phase II clinical trials for non-Hodgkin lymphoma patients (Leonard and Goldenberg, 2007). Similarly to SLE treatments, it is unclear by which mode of action Epratuzumab affects B cell lymphoma cells *in vivo.* While another B-cell specific antibody, Rituximab (anti-CD20), leads to B cell depletion, this has not been demonstrated for anti-CD22 antibodies.

Despite the fact that a lot of effort has been invested into identifying therapeutics based on anti-CD22 antibodies, a suitable model for testing and further investigating the function of antibodies against human CD22 and antibody-coupled immunotoxins is not available so far. Usually, such tests are carried out in non-human animals prior to clinical studies involving humans. Generally, rodents and in particular mice are used in such pre-clinical studies. In the case of CD22, however, the differences between the sequence of human and e.g. murine CD22 protein amount to approx. 40%. Because of this sequence difference, standard laboratory animals expressing their endogenous CD22 are not suitable as model organisms for the investigation of therapeutics targeting human CD22. Accordingly, there is still a need to provide such a model system.

This need is addressed by the provision of the embodiments characterized in the claims.

Accordingly, the present invention relates to a genetically modified rodent comprising a nucleic acid sequence encoding human CD22, or (a) fragment(s) thereof, in its genome.

The term "rodent", as used herein, relates to an order of mammals and is well known in the art. Non-limiting examples of rodents include mice, rats, squirrels, gerbils, porcupines, beavers, chipmunks and guinea pigs. Preferably, the rodent is a mouse or rat. It will be appreciated by the skilled person that the term "rodent" comprises all developmental stages of the respective animal, including the fetus, the embryo, the new-born as well as the adult rodent animal.

The term "genetically modified rodent", as used herein, refers to a rodent whose naturally occurring genetic make-up has been altered. In accordance with the present invention, such alterations include for example the substitution of naturally occurring nucleic acids within the genome of the rodent by a nucleic acid sequence encoding human CD22, or (a) fragment(s) thereof, as well as the addition of a nucleic acid sequence encoding human CD22, or (a) fragment(s) thereof, to the genome of the rodent.
It will be appreciated by the skilled person that the term "genetically modified rodent comprising a nucleic acid sequence encoding human CD22, or (a) fragment(s) thereof, in its genome" refers to a rodent capable of expressing human CD22, or (a) fragment(s) thereof,, i.e. the nucleic acid sequence encoding human CD22, or (a) fragment(s) thereof, is introduced such that it can be transcribed and translated into the corresponding (functional) human CD22 protein or a chimeric rodent-human CD22 protein comprising one or more fragments of human CD22. It will further be appreciated that the introduction of nucleic acid sequence encoding a chimeric rodent-human CD22 protein will also result in the generation of a rodent encompassed by the term "genetically modified rodent comprising a nucleic acid sequence encoding human CD22, or (a) fragment(s) thereof, in its genome".
It is further preferred that where only (a) fragment(s) of human CD22 is introduced into the genome of the rodent, this fragment or these fragments replace the corresponding fragment(s) of the endogenous rodent CD22 sequence. For example, if only the extracellular domain of human CD22 is to be introduced, it is preferred that this human extracellular domain of CD22 replaces the rodent extracellular domain of CD22 while the remainder of the rodent CD22 is not altered.

A naturally occurring nucleic acid is considered to have been substituted within the genome of a rodent if at least one nucleotide of the genome of the rodent is replaced by the nucleic acid sequence encoding human CD22, or (a) fragment(s) thereof. Preferably, more than one nucleotide is replaced, such as for example a part of a gene, e.g. parts of the coding sequence such as one or more exons or parts of exons, as shown in the appended examples. Alternatively, essentially the entire coding sequence for the rodent CD22 may be replaced by the human CD22-encoding nucleic acid sequence. It will be appreciated by the skilled person that also more than the coding sequence of the rodent CD22 locus may be substituted for a corresponding human CD22 sequence, for example comprising regulatory elements as described elsewhere herein.
Addition of a nucleic acid sequence refers to the inclusion of said nucleic acid sequence into the rodents' genome, without the removal of any endogenous sequences by the scientist.

A number of different strategies are known in the art for the modification of the genome of a rodent, all of which may be employed in accordance with the present invention. For example, the nucleic acid sequence encoding human CD22, or (a) fragment(s) thereof, can be introduced into the genome as a transgene, by homologous recombination (HR) techniques for targeted gene modifications or by the use of gene trapping.

For the generation of traditional transgenic animals (Wilmut et al., 1991), the nucleic acid sequence encoding human CD22, or (a) fragment(s) thereof, or a vector comprising this nucleic acid sequence encoding is injected into fertilized eggs of the respective rodent. Embryos are implanted into the uterus of a surrogate mother and the nucleic acid sequence encoding human CD22, or (a) fragment(s) thereof, will be expressed by some of the offspring. This conventional transgenic approach offers the advantage of being relatively straightforward and inexpensive while high levels of transgenic gene expression can be achieved. With this method, the site of integration is random, thus not allowing for a targeted modification of the genome. Consequently, expression of the nucleic acid sequence encoding human CD22, or (a) fragment(s) thereof, can occur in parallel to the expression of the endogenous CD22 gene of the rodent.

In addition to transgenic approaches, gene modification via homologous recombination is also possible. The human CD22 allele, or (a) fragment(s) thereof, is initially assembled in a specifically designed gene targeting vector such that the sequence to be inserted is flanked at both sides with genomic segments of the rodent target sequence that serve as homology regions to initiate homologous recombination. Typically, ES cells are then transfected with the gene targeting vector and recombinant ES cell clones are isolated and subsequently injected into blastocysts to transmit the mutant allele through the germ line of chimeras and to establish a mutant strain. (Hasty P, Abuin A, Bradley A., 2000, In Gene Targeting: a practical approach, ed. AL Joyner, pp. 1-35. Oxford: Oxford University Press; Nagy A, Gertsenstein M, Vintersten K, Behringer R., 2003. Manipulating the Mouse Embryo. Cold Spring Harbour, New York: Cold Spring Harbour Laboratory Press).

To study gene function only in specific cell types or at specific developmental stages, conditional mutants may be generated.
When the aim is conditional inactivation of a target gene, such as may be envisaged in the present case with regard to the rodent CD22 gene, this is achieved by the insertion of two recombinase recognition sites (RRS) for a site-specific DNA recombinase into introns of the target gene or genomic sequence such that recombination results in the deletion of the RRS-flanked exons.
Where the aim is conditional gene activation, the nucleic acid sequence encoding the gene to be activated, i.e. in the present case human CD22, or (a) fragment(s) thereof, comprises a further sequence, such as a gene encoding a resistance marker, flanked by two RRS. For conditional gene activation, the presence of this additional sequence is arranged such that it prevents expression of the gene to be activated, for example by frame-shift or the presence of a polyadenylation signal. Recombination then results in the deletion of the RRS-flanked additional sequence, thus resulting in the expression of human CD22, or (a) fragment(s) thereof, in the cells/tissues expressing the recombinase. Conditional mutants require the generation of two transgenic strains: one strain harboring an RRS flanked segment as described above, obtained by gene targeting in ES cells and a second, transgenic strain expressing the corresponding recombinase in one or several cell types. The conditional mutant is generated by crossing these two strains such that target gene inactivation or activation occurs in a spatial and temporal restricted manner, according to the pattern of recombinase expression in the second transgenic strain (Nagy A, Gertsenstein M, Vintersten K, Behringer R. 2003. Manipulating the Mouse Embryo, third edition ed. Cold Spring Harbour, New York: Cold Spring Harbour Laboratory Press; Torres RM, Kühn R. 1997. Laboratory protocols for conditional gene targeting. Oxford: Oxford University Press).

Gene trapping is a further approach that is used to achieve insertion of nucleic acid sequences of interest into pre-characterised loci in the genome of cells, as a high-throughput method. Exemplary gene trapping techniques that have been developed are based on the replacement of a cassette that was previously introduced into a gene locus, such as e.g. the recombinase mediated cassette exchange (RMCE) as described in Baer and Bode 2001 (J. Curr Opin Biotechnol. 2001; 12(5)) or Flp-RMCE as described in Cesari et al. 2004 (Genesis 2004; 38(2)).

Preferably, the nucleic acid sequence encoding human CD22, or (a) fragment(s) thereof, is inserted into the genome of a recipient rodent by homologous recombination, more preferably by homologous recombination into the locus of the endogenous CD22 gene of the rodent.

The nucleic acid sequence encoding human CD22, or (a) fragment(s) thereof, may be inserted into the CD22 locus in the genome of the recipient rodent, such that the nucleic acid sequence encoding human CD22, or (a) fragment(s) thereof, is under the control of the endogenous regulatory sequences present in the genome of the rodent.

Alternatively, the nucleic acid sequence encoding human CD22, or (a) fragment(s) thereof, may be inserted into the genome of the rodent together with regulatory sequences required to ensure expression of the human CD22 or of chimeric rodent-human CD22. Preferably, the nucleic acid sequence encoding human CD22, or (a) fragment(s) thereof, is operatively linked to such expression control sequences allowing expression in rodent cells. Such regulatory sequences are well known to those skilled in the art and include, without being limiting, regulatory sequences ensuring the initiation of transcription, internal ribosomal entry sites (IRES) (Owens, Proc. Natl. Acad. Sci. USA 98 (2001), 1471-1476) and optionally regulatory elements ensuring termination of transcription and stabilization of the transcript. Non-limiting examples for regulatory elements ensuring the initiation of transcription comprise a translation initiation codon, enhancers such as e.g. the SV40-enhancer, insulators and/or promoters, such as for example the cytomegalovirus (CMV) promoter, SV40-promoter, RSV-promoter (Rous sarcome virus), the lacZ promoter, the gai10 promoter, human elongation factor 1α-promoter, CMV enhancer, CaM-kinase promoter or the autographa californica multiple nuclear polyhedrosis virus (AcMNPV) polyhedral promoter but also the human CD22 promoter or the respective rodent, e.g. mouse, CD22 promoter. Non-limiting examples for regulatory elements ensuring transcription termination include the V40-poly-A site, the tk-poly-A site or the SV40, lacZ or AcMNPV polyhedral polyadenylation signals, which are to be included downstream of the nucleic acid sequence encoding human CD22. Additional regulatory elements may include translational enhancers, nucleotide sequences encoding secretion signals or, depending on the expression system used, signal sequences capable of directing the expressed polypeptide to a cellular compartment. All of the above described sequences are well known in the art.

The nucleic acid sequence encoding human CD22, or (a) fragment(s) thereof, may furthermore be inserted into the genome of the rodent together with a selectable marker. Examples of selectable markers include markers providing resistance to neomycin, ampicillin, hygromycin and the like and are well known in the art.

The nucleic acid sequences for generation of the genetically modified rodent may e.g. be designed for direct introduction or for introduction via electroporation (using for example Multiporator (Eppendorf) or Genepulser (BioRad)), PEI (Polysciences Inc. Warrington, Eppelheim), Ca²⁺-mediated transfection or via liposomes (for example: "Lipofectamine" (Invitrogen)), non-liposomal compounds (for example: "Fugene" (Roche)), liposomes, phage vectors or viral vectors (e.g. adenoviral, retroviral, lentiviral) into cells. Additionally, baculoviral systems or systems based on Vaccinia Virus or Semliki Forest Virus can also be used.

Nucleic acid sequences, in accordance with the present invention, include DNA, such as cDNA or genomic DNA including exonic and intronic sequences. Preferably, the nucleic acid sequence encoding human CD22 is cDNA, or (a) fragment(s) thereof,.

CD22 refers to cluster of differentiation-22 and is well known to the person skilled in the art. CD22 is a member of the Siglec family (Sialic-acid binding immunoglobulin-like lectins). The members of the Siglec family are adhesion molecules that specifically bind to sialic acids, which are usually expressed on cell surfaces and in the extracellular milieu (Crocker et al., 2007; Crocker and Redelinghuys, 2008). All Siglecs recognize neuraminic acids (Neu5Ac), but they vary in their affinity for different glycoside-linkages. CD22 prefers sialic acids in α2,6-linkage (Nitschke, 2009; Walker and Smith, 2008). The human CD22 gene is located on chromosome 19 in the human genome, while the mouse gene is located on chromosome 7. Human CD22 is for example represented by the UniProt Accession number P20273 or the RefSeq ID NP_001762 and its mRNA is represented by RefSeq ID NM_001771(Wilson et al., 1993). The cDNA encoding human CD22 is shown in SEQ ID NO:1.

The term "fragment of human CD22", as used herein, refers to a part of the full-length nucleic acid sequence encoding human CD22. Such fragments include nucleic acid sequence in which a given number of nucleotides at the 3' or 5' end have been removed. Additionally or alternatively, a number of internal (non-terminal) nucleotides may be removed, provided the resulting nucleic acid sequence still encodes a part of, or the entire, human CD22. In particular, removals of nucleotides which preserve sequence and boundaries of functional domain(s) or subsequences in human CD22 are particularly envisaged, such as e.g. preserving the extracellular domain or parts thereof, such as one or more of the Ig-like domains. Such domains of CD22 are well known in the art or may be determined by means and methods known to the skilled person. Such methods include experimental and bioinformatic means. Experimental means include the systematic generation of deletion mutants and their assessment in assays for CD22 activity known in the art and as described elsewhere herein. Bioinformatic means include database searches. Suitable databases included protein sequence databases. In this case a multiple sequence alignment of significant hits is indicative of domain boundaries, wherein the domain(s) is/are comprised of the/those subsequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam).
Preferable sizes of fragments in accordance with the present invention are at least 10 nucleotides, such as at least 50 nucleotides, such as at least 100 nucleotides, such as at least 200 nucleotides, such as at least 300 nucleotides, such as at least 400 nucleotides, such as at least 500 nucleotides, such as at least 600 nucleotides, and more preferably at least 700 nucleotides. Most preferably, the fragment consists of or comprises the 710 amino acids encoding the extracellular domain of human CD22. Also envisaged herein are fragments having a size of at least 1000 nucleotides, such as at least 1500 nucleotides, such as at least 2000 nucleotides, such as at least 2500 nucleotides, or such as at least 3000 nucleotides.
It will be appreciated by the skilled person that fragments resulting in a nucleic acid sequence having an identical sequence to the corresponding fragment of the endogenous rodent CD22 gene are not encompassed by the term "fragment of human CD22" in accordance with the present invention.
The term "(a) fragment(s)", as used herein, encompasses a single fragment as well as a plurality of fragments, such as e.g. two, three, four or more fragments.

In accordance with the present invention, it was surprisingly found that human CD22 can replace murine CD22 without the occurrence of any defects in B cell maturation. The genetically modified animals showed normal B cell development, similar to wild type animals. Both the populations of precursor B cells in the bone marrow, as well as mature B cells in spleen and lymph node developed in normal numbers. This normal B cell maturation is surprising, as the human CD22 and murine CD22 have only 60% sequence similarity. For example, the intracellular domains containing the 6 tyrosine motifs, including 3 ITIM motifs, have a similarly distinct sequence between human and murine CD22. Thus, intracellular signalling proteins, which bind to the intracellular domain of mouse CD22 appear to be able to also bind to the human CD22 intracellular domain and, consequently, permit normal B cell maturation. Also the ligand specificity of human and murine CD22 is different. While the murine protein prefers sialic acids containing Neu5Gc, the human protein binds to both Neu5Gc, as well as Neu5Ac (Kelm et al.1994). The mouse only expresses the Neu5Gc form of sialic acids, however, due to the dual specificity of human CD22 normal differentiation of B cells expressing human CD22 is nevertheless possible.

As is shown in the appended examples, human knock-in (Huki) CD22-transgenic mice were generated that express human CD22 instead of murine CD22. This mouse line was generated by knock-in of the human CD22 cDNA into the mouse Cd22 gene. As a result, human CD22 protein is expressed under the control of the murine Cd22 promoter and, therefore, is transcriptionally regulated in the same manner as the endogenous mouse Cd22 gene. The homozygous mice were analyzed by flow cytometry, showing that the B-cells express human but not murine CD22.

This mouse model can serve as a valuable model for testing the biological effects of e.g. anti-human CD22 antibodies and anti-CD22 immunotoxins, such as those that are already in clinical studies for the treatment of lymphoma and SLE patients. Similar models expressing only a fragment of CD22, for example chimeric CD22 having the human extracellular domain and a rodent intracellular domain, can also be employed in such tests.

In a preferred embodiment of the genetically modified rodent of the invention, the human CD22, or (a) fragment(s) thereof, is expressed on the surface of B lymphocytes.

"B lymphocytes" are well known in the art and are characterised by the expression of surface markers, for example B220, CD19, CD22, IgM, IgD, CD23 and B lymphocytes, also referred to as B cells herein, play a major role in the humoral immune response, as they produce antibodies against antigens, act as antigen-presenting cells (APCs) and can develop into memory B cells after activation by antigen interaction. B lymphocytes are an essential component of the adaptive immune system. However, dysregulated B lymphocytes can also contribute to the pathogenesis of diseases, such as for example autoimmune diseases like systemic lupus erythematosus (SLE) (Dorner et al., 2009).

Means and methods to verify that human CD22, or (a) fragment(s) thereof, is expressed on the surface of B lymphocytes in the genetically modified rodent are known to the skilled person. For example, B lymphocytes may be isolated using a mouse B lymphocyte-specific marker and then analysed for the presence of human CD22, or (a) fragment(s) thereof, e.g. by staining using human CD22-specific antibodies. Alternatively, cells may be analysed using flow cytometry, as described in the examples below.

In another preferred embodiment of the genetically modified rodent of the invention, the expression of human CD22, or (a) fragment(s) thereof, is under the control of the endogenous rodent CD22 promoter.

The term "endogenous rodent CD22 promoter" relates to the promoter that controls the expression of CD22 in the respective rodent. For example, when the rodent is mouse, the endogenous rodent CD22 promoter is the mouse CD22 promoter.

Methods to ensure that the human CD22, or (a) fragment(s) thereof, is controlled by the respective rodent promoter include for example the targeted integration of the nucleic acid sequence encoding the human CD22, or (a) fragment(s) thereof, 3' of the endogenous rodent promoter. Means to ensure correct integration of a nucleic acid sequence downstream of a promoter in order to obtain expression of said nucleic acid sequence under the control of the promoter are well known in the art and include, without being limiting, integration of the nucleic acid sequence in a short distance (e.g. 10bp to 100bp) downstream of the promoter and use of an optimal translational start site (Kozak sequence). Alternatively, the rodent promoter may be operatively linked to the nucleic acid sequence encoding human CD22, for example by generating a vector comprising both the CD22 sequence as well as the promoter sequence and introducing said vector into the genome of the rodent.

In a further preferred embodiment of the genetically modified rodent of the invention, the expression of the endogenous nucleic acid sequence encoding the rodent CD22 is disrupted.

The disruption in the expression of the endogenous nucleic acid sequence encoding rodent CD22 may be achieved, for example, by homologous recombination replacing the entire endogenous nucleic acid sequence encoding CD22 with the corresponding nucleic acid sequence encoding human CD22. A disruption may also be achieved by incorporating the nucleic acid sequence encoding human CD22 into the rodent CD22 locus. Where said incorporation leads to a frame shift within the coding regions of the endogenous gene, this can be sufficient to lead to a disruption in the expression of rodent CD22. Alternatively, the nucleic acid sequence encoding human CD22 may comprise a stop codon and, optionally, a polyadenylation sequence, thus terminating expression of CD22 at the 3' end of the human nucleic acid sequence, as shown in the appended examples.

In accordance with this embodiment, expression of the rodent CD22 no longer occurs and is replaced by the expression of human CD22. All B cells express the same amount of human CD22, without possible variations in the relative level of human and murine CD22. Thus, effects achieved with compounds directed against CD22 can be ascribed to an effect of the compounds on human CD22, without any interference of the rodent CD22 protein. In addition, it is possible to investigate the role of CD22 ligands since human CD22 and murine CD22 have different ligand specificity, as discussed above (Kelm et al., 1994)

In another preferred embodiment of the genetically modified rodent of the invention, the rodent is a mouse. More preferably, the mouse is selected from the group consisting of C57BL/6, BALB/c, 129/Sv and 129/Ola.

In a more preferred embodiment of the genetically modified mouse, the nucleic acid sequence encoding human CD22 is cDNA and is integrated into an exon 3' of the signal peptide sequence of the endogenous murine CD22 gene.

Human CD22 cDNA has previously been described in Wilson et al., 1993 and is for example represented by the RefSeq ID ENST00000085219 and is shown for example in SEQ ID NO:1. The nucleic acid sequence encoding mouse CD22 is shown in SEQ ID NO:3.

The term "signal peptide", as used herein, refers to a short peptide chain that directs the transport of a protein. Signal peptides are also referred to in the art as targeting signals, signal sequences, transit peptides, or localization signals. The "signal peptide sequence of the endogenous murine CD22 gene" is encoded in exon 3 and the first part of exon 4 of the endogenous murine CD22 gene and is shown in SEQ ID NO: 2.

Due to the integration of the cDNA encoding human CD22 in a position 3' of the signal peptide sequence (i.e. into exon 4 of the murine CD22 gene as shown in the examples below), the correct transport of the translated human CD22 protein to the cell surface is ensured.

The present invention further relates to tissue and/or germ cells obtained from the genetically modified rodent of the invention, wherein the tissue or germ cells comprise the genetic modification, i.e. the tissue or germ cells comprise a nucleic acid sequence encoding human CD22, or (a) fragment(s) thereof, in their genome and are capable of expressing functional human CD22 protein or a functional chimeric rodent-human CD22 protein.

Methods for testing whether a human CD22 protein or a chimeric rodent-human CD22 protein is functional are well known in the art and include, without being limiting, analysing the binding to α2,6-linked sialic acid-containing ligands, the inhibition of BCR-induced Ca²⁺ signalling, flow cytometry analysis of CD22 expression on the cell surface of B cell populations, analysis of the distribution of CD22 positive B cells by histology, analysis of the downstream signalling of CD22 by e.g. western blot analysis, immunoprecipitation, analysis of tyrosine phosphorylation and the recruitment of tyrosine phosphatase SHP-1

The term "tissue", as used herein, relates to biological materials obtained from an animal, which comprises a plurality of (identical or different) cells. Non-limiting examples of tissues are lymph nodes, spleen, thymus, bone marrow, blood vessels, liver or kidney as well as portions thereof. Such tissues may be useful in *in vitro* experimental tests for testing compounds directed towards the human CD22 protein.

The term "germ cell" relates to cells that gives rise to the gametes of an organism that reproduces sexually. The germ cells migrate to the developing gonads, where they undergo mitosis and meiosis, followed by cellular differentiation into mature gametes, namely either eggs or sperm. The germ cells obtained from the genetically modified rodent of the invention may for example be employed to generate further offspring of the genetically modified rodents of the invention.

The present invention further relates to a method of identifying an agent capable of treating diseases associated with dysregulated B lymphocytes, the method comprising: (a) administering an agent to the genetically modified rodent of the invention; and (b) determining the amount and/or activity of B lymphocytes expressing human CD22, or (a) fragment(s) thereof, in the rodent after administration of the agent in step (a); wherein a decrease in the amount and/or activity of B lymphocytes determined in step (b) as compared to the amount and/or activity of B lymphocytes prior to the administration of the agent in step (a) is indicative of an agent capable of treating diseases associated with dysregulated B lymphocytes.

The agent to be tested according to this method of the invention may, for example, be a small molecule, an antibody or a fragment or derivative thereof, an aptamer, an siRNA, an shRNA, a miRNA, a ribozyme or an antisense nucleic acid molecule.

A "small molecule" according to the present invention may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in *in vivo* assays such as in vivo high-throughput screening (HTS) assays.

The term "antibody" as used in accordance with the present invention comprises, for example, polyclonal or monoclonal antibodies. Furthermore, also derivatives or fragments thereof, which still retain the binding specificity, are comprised in the term "antibody". Such fragments or derivatives comprise, inter alia, Fab or Fab' fragments as well as Fd, F(ab')2, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), loc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), loc. cit. and include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of STIM2 or an epitope of a STIM2-regulated plasma membrane calcium channel (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold. Nucleic acid aptamers are nucleic acid species that, as a rule, have been engineered through repeated rounds of in vitro selection or equivalently, SELEX (systematic evolution of ligands by exponential enrichment) to bind to various molecular targets such as small molecules, proteins, nucleic acids, and even cells, tissues and organisms. Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a - Cys-Gly-Pro-Cys- loop in the wild protein, the two cysteins lateral chains being able to form a disulfide bridge. Peptide aptamer selection can be made using different systems, but the most widely used is currently the yeast two-hybrid system.

Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of inherently low molecular weight of aptamers. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "protein" as used herein describes a group of molecules consisting of more than 30 amino acids. Peptides and proteins may further form dimers, trimers and higher oligomers, i.e. consisting of more than one molecule which may be identical or non-identical. The corresponding higher order structures are, consequently, termed homo- or heterodimers, homo- or heterotrimers etc. The terms "peptide" and "protein" (wherein "protein" is interchangeably used with "polypeptide") also refer to naturally modified peptides/proteins wherein the modification is effected e.g. by glycosylation, acetylation, phosphorylation and the like. Such modifications are well-known in the art.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.+

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'- overhang. The sequence of the 2-nt 3' overhang makes a small contribution to the specificity of target recognition restricted to the unpaired nucleotide adjacent to the first base pair (Elbashir et al. 2001 ). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery *in vivo* through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics).

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed as an inhibitor of gene expression.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and in vitro-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

It will be appreciated by the skilled person that the agent identified by this method often represents a lead compound which may require further optimization, for example in order to be pharmaceutically more acceptable. Methods for the optimization of the pharmacological properties of agents identified in such screens are known in the art and comprise a method of modifying an agent identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiffs bases, oximes, acetales, ketales, enolesters, oxazolidines, thiazolidines or combinations thereof.

The various steps recited above are generally known in the art. They include or rely on quantitative structure-activity relationship (QSAR) analyses (Kubinyi (1992) "Hausch-Analysis and Related Approaches", VCH Verlag, Weinheim), combinatorial biochemistry, classical chemistry and others (see, for example, Holzgrabe and Bechtold (2000) Deutsche Apotheker Zeitung 140(8), 813).

Methods for administering an agent to a rodent are well known in the art and include, without being limiting, oral, rectal, parenteral, intracisternal, intraperitoneal, topical (e.g. as powders, ointments, drops or transdermal patch), buccal administration. Administration may also be via continuous subcutaneous infusions, for example, using a mini-pump or employing an intravenous bag solution.

The amount of B lymphocytes is considered to be decreased if it is reduced to at least 90% of the amount of B lymphocytes prior to the administration of the agent in step (a), more preferably to at least 80%, such as at least 70%, at least 60%, at least 50%, at least 40%, at least 30%, at least 20%, more preferably at least 10% and most preferably 0% of the amount of B lymphocytes prior to the administration of the agent in step (a).

Similarly, the activity of B lymphocytes is considered to be decreased if it is reduced to at least 90% of the activity of B lymphocytes prior to the administration of the agent in step (a), more preferably to at least 80%, such as at least 70%, at least 60%, at least 50%, at least 40%, at least 30%, at least 20%, more preferably at least 10% and most preferably 0% of the activity of B lymphocytes prior to the administration of the agent in step (a).

In accordance with this embodiment, the agent to be tested is administered to the rodent and the amount and/or activity of B lymphocytes expressing human CD22, or (a) fragment(s) thereof, in the rodent is determined. The determination of the amount of B lymphocytes is well known in the art and, preferably, is carried out by FACS analysis. Methods for determining the activity of B lymphocytes are also known in the art and include, without being limiting, analysis of antibody production by ELISA and *in vitro* analysis of B cell functionality in cell culture, for example by testing their ability to be activated by treatment such as anti-IgM, LPS or anti-CD40. The appropriate time point after administration to determine the amount and/or activity of lymphocytes depends on the agent employed and can be determined by the skilled person without further ado. Preferably, the amount and/or activity of B lymphocytes is determined between 10 minutes to 100 hours after administration, such as e.g. between 30 minutes to 90 hours, more preferably between 1 hour and 80 hours, such as between 2 and 72 hours, such as between 3 hours and 48 hours, such as between 4 and 36 hours, and most preferably between 6 and 24 hours after administration of the agent to the rodents.

The amount and/or activity of B lymphocytes expressing human CD22, or (a) fragment(s) thereof, thus determined is then compared to the amount of B lymphocytes expressing human CD22, or (a) fragment(s) thereof, prior to administration of the agent. It will be appreciated that the amount of B lymphocytes expressing human CD22, or (a) fragment(s) thereof, prior to administration of the agent can be determined each time before carrying out the method of the invention or, alternatively, a basal value in the rodent employed in the method of the invention may be established once and used as a reference value. Preferably, the amount of B lymphocytes expressing human CD22, or (a) fragment(s) thereof, is determined each time prior to administration of the agent.

If the amount and/or activity of B lymphocytes is decreased after administration of the agent as compared to the amount and/or activity of B lymphocytes prior to the administration of the agent, then it can be concluded that the agent is capable of treating diseases associated with dysregulated B lymphocytes.

In a preferred embodiment of the method of identifying an agent capable of treating diseases associated with dysregulated B lymphocytes, the diseases associated with dysregulated B lymphocytes are selected from the group consisting of autoimmune diseases, cancer and common variable immune deficiency (CVID).

The term "autoimmune disease", in accordance with the present invention, refers to diseases which arise from overactive T-cell mediated immune responses of the body against substances and tissues normally present in the body as well as over-reactive B cells and autoantibody production (Townsend et al., 2010).

"Cancer", in accordance with the present invention, refers to a class of diseases or disorders characterized by uncontrolled division of cells and the ability of these to spread, either by direct growth into adjacent tissue through invasion, or by implantation into distant sites by metastasis, where cancer cells are transported through the bloodstream or lymphatic system.

"Common variable immune deficiency (CVID)", in accordance with the present invention, refers to a disorder characterized by low levels of serum immunoglobulins (antibodies) and an increased susceptibility to infections. In some patients, there is a decrease in both IgG and IgA while in others, all three major types (IgG, IgA and IgM) of immunoglobulins can be decreased.

In a more preferred embodiment of the method of identifying an agent capable of treating diseases associated with dysregulated B lymphocytes, the autoimmune disease is selected from the group consisting of systemic lupus erythematosus (SLE), Rheumatoid arthritis, Goodpasture Syndrome and Multiple Sclerosis.

Systemic lupus erythematosus refers to a chronic (long-lasting) autoimmune disease wherein the immune system becomes hyperactive and attacks normal tissue, resulting in inflammation. Systemic lupus erythematosus is a "non-organ-specific" type of autoimmune disease (Pineles et al., 2011).

Rheumatoid arthritis is an autoimmune disorder that causes the body's immune system to attack the bone joints (Muller B et al. 1998. Springer Semin Immunopathol. 20:181-96). Rheumatoid arthritis is a chronic, systemic inflammatory disorder that may affect many tissues and organs, but principally attacks synovial joints. The pathology of the disease process often leads to the destruction of articular cartilage and ankylosis of the joints. Rheumatoid arthritis can also produce diffuse inflammation in the lungs, pericardium, pleura, and sclera, and also nodular lesions, most common in subcutaneous tissue under the skin.

Goodpasture Syndrome is an autoimmune disease in which the immune system attacks Goodpasture antigen (a type II hypersensitivity reaction), which is found in the kidney and lung, thus resulting in damage to these organs and causing glomerulonephritis and hemorrhaging of the lungs (Bolton et al., 2005).

Multiple sclerosis is an autoimmune disease in which the immune system attacks the central nervous system (CNS), leading to the demyelination of neurons (Shevach EM et al. 1991. Springer Semin Immunopathol. 21:249-62; Leonard JP et al. 1997. Crit Rev Immunol. 545-53).

In another more preferred embodiment of the method of identifying an agent capable of treating diseases associated with dysregulated B lymphocytes, the cancer is selected from the group consisting of B-cell lymphomas and B-cell leukemias.

The term "B-cell lymphomas" are cancers of the blood in the lymph glands and affect B cells. Examples of B-cell lymphomas include diffuse large B cell lymphoma, follicular lymphoma, mucosa-associated lymphatic tissue lymphoma (MALT), small cell lymphocytic lymphoma, mantle cell lymphoma (MCL), burkitt lymphoma, mediastinal large B cell lymphoma, waldenström macroglobulinemia, nodal marginal zone B cell lymphoma (NMZL), splenic marginal zone lymphoma (SMZL), intravascular large B-cell lymphoma, primary effusion lymphoma or lymphomatoid granulomatosis (Kurtin 2009).

The term "B-cell leukemias" describes lymphoid leukemias which affect B cells. Lymphoid leukemia relates to blood cancer affecting circulating lymphocyte cells, in contrast to lymphoma, which is a solid tumor of the same type of cells. Examples of B-cell leukemias include B-cell chronic lymphocytic leukemia, acute lymphoblastic leukemia of the mature B-cell type, precursor B lymphoblastic leukemia, B-cell prolymphocytic leukemia or hairy cell leukemia (Szczepañ et al. 2003).

The rodent model of the present invention can furthermore be crossed to disease models, for example SLE models known in the art, such as Fcgamma Rllb-/- mice, MRL Ipr/Ipr mice or NZB/NZW mice. Autoantibodies, for instance against DNA, RNA, nuclear antigens, chromatin and other self antigens, taken from serum of these mouse strains can be determined prior to and after treatment with the agent to be tested. Alternatively, or additionally, further parameters can be determined such as e.g. protein urea, kidney pathology and survival of the mice.
Accordingly, the present invention further relates to a method of identifying an agent capable of treating autoimmune diseases associated with dysregulated B lymphocytes, the method comprising: (a) administering an agent to a rodent animal model obtained from mating the genetically modified rodent of the invention with a disease model; and (b) determining the amount of disease-specific autoantibodies in the rodent after administration of the agent in step (a); wherein a decrease in the amount of disease-specific autoantibodies determined in step (b) as compared to the amount of disease-specific autoantibodies prior to the administration of the agent in step (a) is indicative of an agent capable of treating autoimmune diseases associated with dysregulated B lymphocytes.

All the definitions and preferred embodiments described herein with regard to the method of identifying an agent capable of treating diseases associated with dysregulated B lymphocytes apply mutatis mutandis also to this embodiment. Moreover, it is within the skills of the skilled person to determine the relevant disease-specific autoantibodies for the autoimmune model of interest. Such autoantibodies include for example anti-dsDNA (SLE), IgM anti-IgG and anti-cyclic citrullinated peptide (CCP), both in RA, anti-Acetylcholine receptors (Myasthenia gravis) etc. (Townsend, Monroe, Chan Immunol. Rev. 2010 237, 264).

The present invention further relates to a method of testing the safety of an agent for anti-human CD22 therapy, the method comprising: (a) administering the agent to the genetically modified rodent of the invention; and (b) analysing said rodent for short or long term adverse effects.

All the definitions and preferred embodiments described herein with regard to the method of identifying an agent capable of treating diseases associated with dysregulated B lymphocytes apply mutatis mutandis also to this embodiment.

Adverse side effects relate to harmful and undesired effects resulting from treatment with the agent and include, without being limiting, morbidity, mortality, alteration in body weight, pathological changes detected at the microscopic, macroscopic or physiological level, diarrhea, vomiting, somnolence or infections.

In accordance with this embodiment, the safety of an agent, including for example agents identified by the method described herein or agents currently available in the art, can be tested prior to administration to human patients. The occurrence of any adverse effects is indicative of the agent not being safe and, therefore, not suitable for the therapy of human patients.

Unless otherwise defined, all technical and scientific terms used herein have the same meaning as commonly understood by one of ordinary skill in the art to which this invention belongs. In case of conflict, the patent specification, including definitions, will prevail.

The figures show:
**Figure 1**: Targeting of the murine Cd22 gene by a targeting vector, creating a knock-in of the human CD22 cDNA into the mouse Cd22 gene. cDNA: human CD22 cDNA, neo: neomycin cassette, checked pattern: polyA signal, triangle: IoxP site, 1-5: exons of the murine CD22 gene.
**Figure 2**: Targeting vector for HuKi CD22 transgenic mouse.
**Figure 3**: Human CD22 cDNA sequence (black) cloned into the targeting vector; grey: amino acid translation.
**Figure 4**: PCR screening of ES-cell clones. St: standard, -: negative clone, arrow: expected PCR band of the positive clone.
**Figure 5****:** Southern Blot analysis with an external probe over the short arm of the construct; grey: external probe, broken line: flanking DNA outside of the short arm of the vector, neo: neomycin cassette, cDNA: human CD22 cDNA, triangle: IoxP site, arrows: expected size of target allele. For this analysis the ES cell DNA was digested with Pstl.
**Figure 6****:** Southern Blot analysis with an internal probe over the short arm of the construct; grey: internal probe, broken line: DNA outside of the short arm of the vector, arrows: expected size of target allele, neo: neomycin cassette, cDNA: human CD22 cDNA. For this analysis the ES cell DNA was digested with Sacl.
**Figure 7****:** PCR screening of the offspring of the chimeric males. St: standard, +/-: heterozygous mice, +/+: WT mice, arrows: expected sizes of wildtype and "knock-in" bands.
**Figure 8****:** Flow cytometry staining of murine blood cells of the wildtype, heterozygous and homozygous Huki CD22-transgenic mice, gated on B220 positive cells.
**Figure 9****:** Flow cytometry staining of murine bone marrow cells of the wild type and homozygous Huki CD22-transgenic mice, gated on lymphocytes. R1: pro and pre B cells, R2: immature B cells, R3: transitional B cells, R4: mature recirculating B cells. The chart shows the amount of gated cells in the regions in %.
**Figure 10****:** Flow cytometry staining of murine spleen and lymph node cells of the wildtype and homozygous Huki CD22-transgenic mice, gated on lymphocytes.
   A: Staining of the spleen cells shows that Huki (-/-) mice express human CD22 but no murine CD22 on the surface of B220 positive cells. Spleen cells from the wildtype mice express murine CD22 but no human CD22. B cells are found in roughly normal numbers. B: Staining of the lymph node cells shows that Huki (-/-) mice express murine CD22 but no murine CD22 on the surface of the B220 positive cells. B cells are found in roughly normal numbers.

The examples illustrate the invention:

### Example 1: Vector cloning

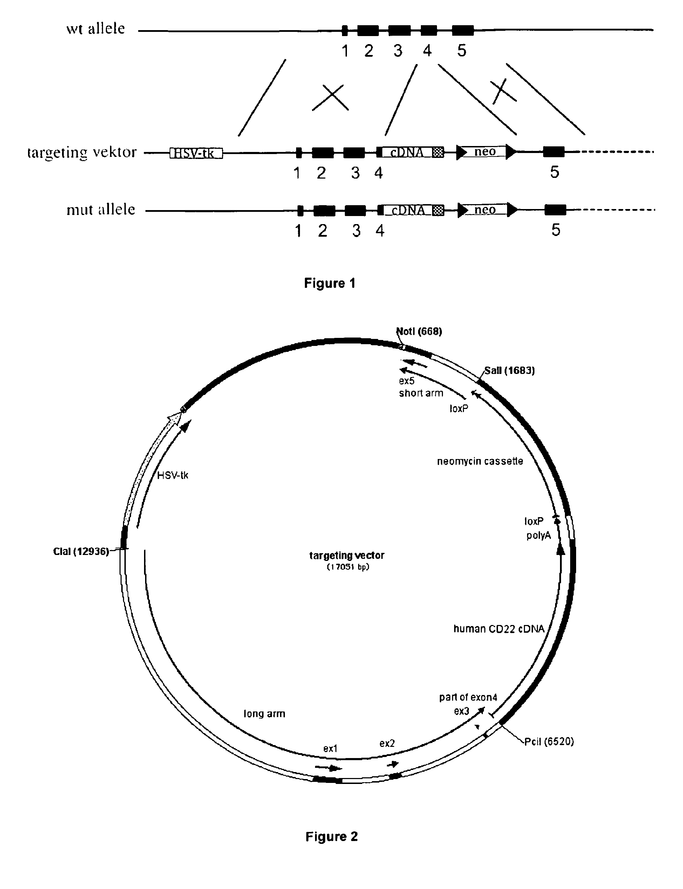

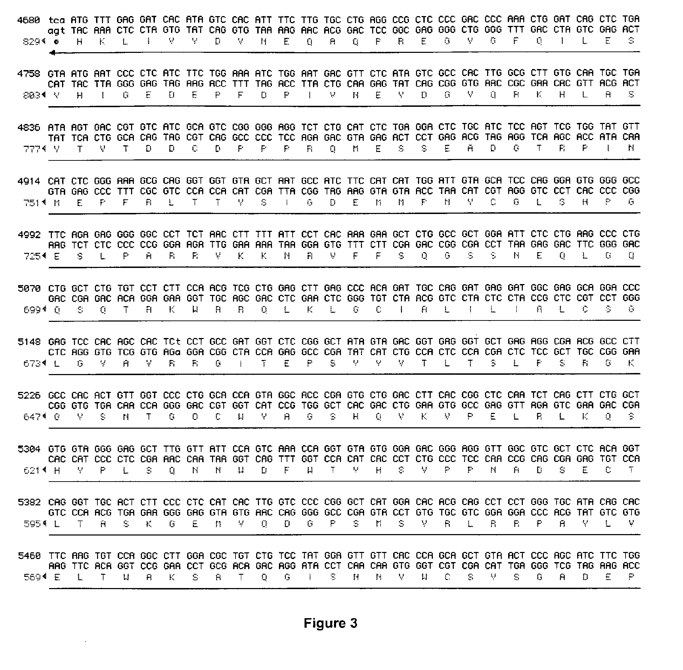

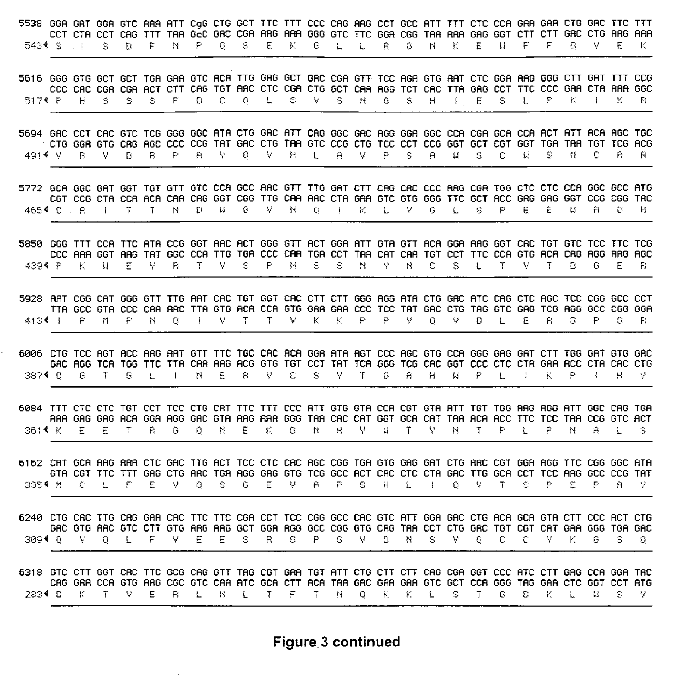

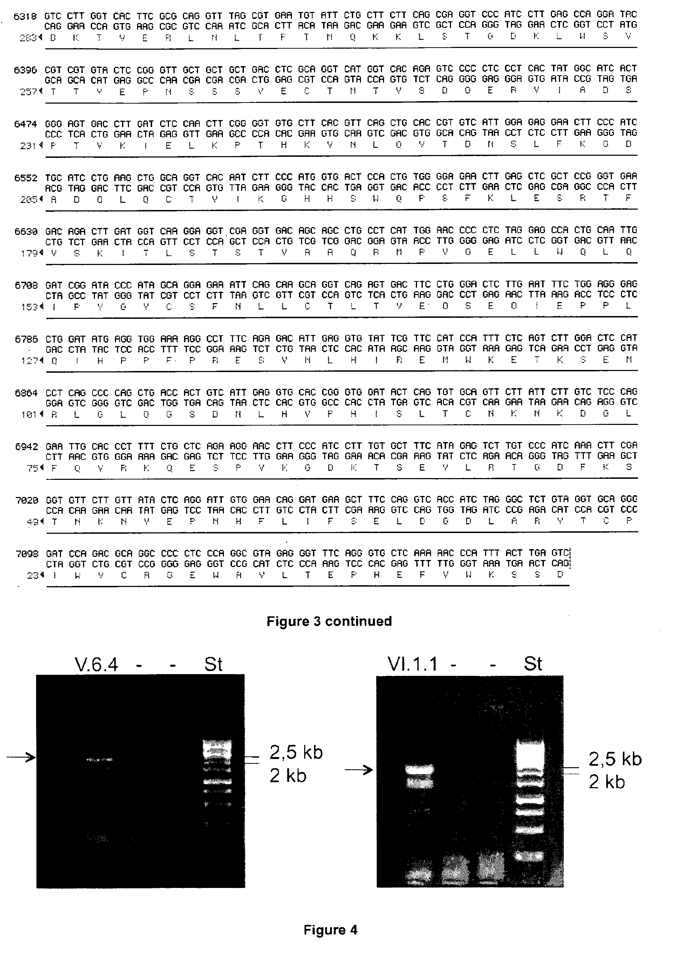

The targeting vector was constructed following the scheme in figure 1. The plasmid map is shown in figure 2. As a first cloning step, the polyA signal was cloned into the vector pKStneoloxP from the vector IRES-eGFP (Clonetech). The polyA signal was amplified by PCR, using the primers polyA-Bsu15lfw (5'-ATATCGATATTCGGACGTGACTGACATATGCGCTCTGTACACCCCCTGAACCTGAAACATAAAATG-3') and polyA-BamHlrev (5'-ATGGATCCACTCAACCCTATCTCGGTC-3'). The PCR fragment and the targeting vector were digested with the enzymes BamHI and Bsu15I and then ligated. The human cDNA was then isolated from human blood cells using the primers cDNA-Pcilrev (5'-TCTGTACATCAATGTTTGAGGATCACATAGTCCAC-3') and cDNA-Bsu15Ifw (5'-ATATCGATA TGCTAAGTAGTGTATTGTAGGACATGTGGCTTCGGCTGACTCAAGTAAATGGGTTTTTGAG-3'). The sequence of the cDNA is shown in figure 3. By digesting the cDNA and the vector with the enzymes Bsp1407I and Bsu15I, the cDNA could be cloned into the targeting vector. The long homology arm of the targeting vector was amplified in two parts. The first part contained the genomic nucleic acid sequence including the murine exons 1 to 3 and part of exon 4 and was obtained from the vector pKSCD22ex1-5R130E (generated in the Nitschke group as described in the diploma thesis of Carolin Geus, Erlangen, 2006).

After digesting this vector and the targeting vector with the enzymes Pscl and Bsu15I, the first part of the long homology arm was cloned into the targeting vector. Then, the second part of the long homology arm containing the promoter sequence, was prepared. A PCR reaction was performed with genomic DNA of ES-cells from the line E14creAG using the primers 22prom-LAfw (5'-TTATCGA TGATGCTGTATGGTACTGG-3') and 22prom-LArev (5'-GCTTTCGCTTGCTGATACATC-3'). As a last step, the short homology arm, containing the DNA of murine exon 5 and flanking intron sequence was cloned into the construct. The vector pKS-R130E-Kontrollplasmid (generated in the Nitschke group as described in the diploma thesis of Carolin Geus, Erlangen, 2006) and the targeting vector were digested with the enzymes Notl and Sall and the short homology arm fragment was cloned into the targeting vector.

The vector pKStneoIoxP, used as a starting vector for the construction of the targeting vector, already contains LoxP sites flanking a neo cassette. The long homology arm in front on the cDNA comprises the promoter region, exons 1 to 3 and a part of exon 4 of the murine CD22 gene. The signal peptide, which is responsible for correct CD22 transport to the cell surface, is encoded in exon 3 and the first part of exon 4, and is therefore maintained. The human cDNA was cloned in frame into exon 4 of the murine CD22 gene, 3' of the sequence coding for the signal peptide.

The targeting vector further comprises a HSV-tk cassette in front of the long arm (see figures 1 and 2). After homologous recombination, the HSV-tk cassette is not present in the genome comprising the recombined sequence and, consequently, ES-cells with successful homologous recombination events can be selected for by addition of ganciclovir to the culture medium.

### Example 2: Transfecting embryonic stem cells with the vector DNA

The targeting vector was opened at the short arm by digestion with the enzyme Notl and the linearised vector was then sterilised by ethanol precipitation. Embryonic stem cells (ES-cells) from the line E14CreAG (129sv background, expressing protamine-Cre; O'Gorman et al., 1997) were transfected with the linearised vector and plated on cell culture dishes, which were covered with a confluent layer of embryonic fibroblasts (EMFIs) as feeder cells. After one day, the ES cell medium (DMEM with 15% FCS and LIF) was replaced with selection medium containing G418 and ganciclovir, enabling the selection of cells having a neo cassette but no HSV-tk cassette and, thus, selecting cells in which homologous recombination occurred at the expected site was successful. Selection with ganciclovir is a negative selection in which the cells survive if they loose the HSV-tk cassette due to homologous recombination in the long homology arm.
After 10 days, 480 clones were picked and DNA of these clones was isolated and screened by PCR. For this screening we used a nested PCR with the Primers 5'- TGA TCC GTG CAC TGG CAT AC -3' and 5'- CGG TAT CGC CGC TCC CGA TT -3' for the first PCR and 5'- ACC TGT GTG CTC AGC CTA AG - 3' and 5'- CGC CTT CTA TCT CCT TCT TGA -3' for the second PCR.
An amplification product of 2400 basepairs was expected for positive clones. Two positive clones - termed V.6.4 and V1.1.1 - out of 480 clones were detected in repeated PCR assays (figure 4).

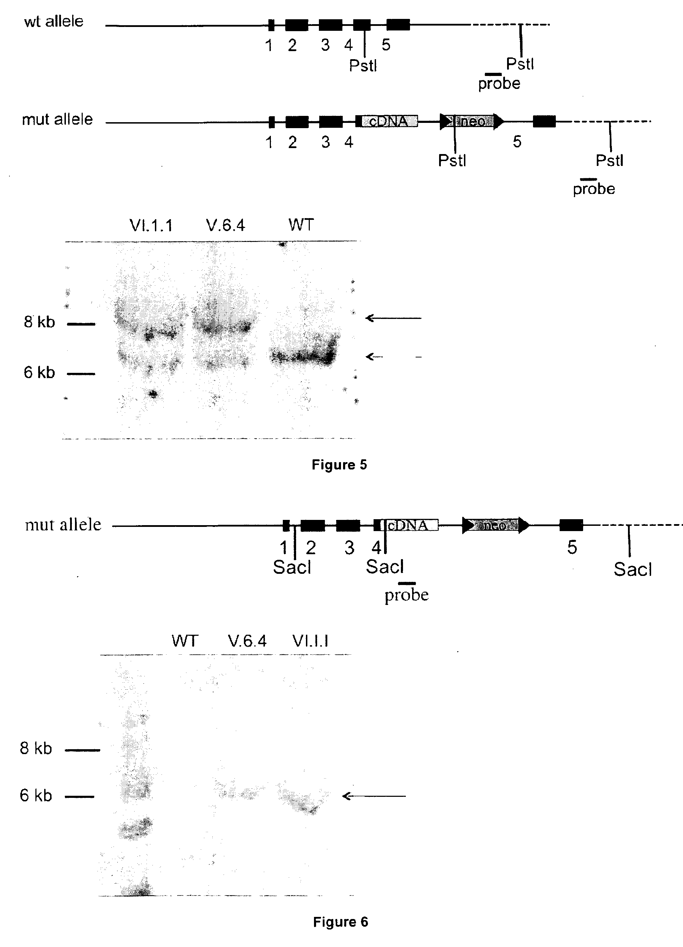

Southern blot analyses over both the long and the short arm was employed to confirm the correct insertion of the human CD22 sequence in these two positive clones. For the Southern Blot analysis over the short arm, DNA was digested with the enzyme Pstl and detected with an external probe (see figure 5). The wild type band was expected at 6759 basepairs, while the knock-in band was expected at 7985 basepairs. A second Southern Blot over the short arm of the targeting vector with an internal probe was performed by digesting the different ES cell DNA with Sacl and detecting the knock-in band with a human CD22 cDNA specific probe (figure 6). The expected band was 6600 basepairs long.
In order to confirm the correct insertion further, a Southern Blot analysis over the long homology arm of the construct was performed. The DNA of the ES-cell clones was digested with the enzyme Sail and subsequently detected with either an external or internal probe. Also these experiments confirmed the correct integration of the human CD22 cDNA.

### Example 3: Generation of a knock-in mouse carrying human CD22

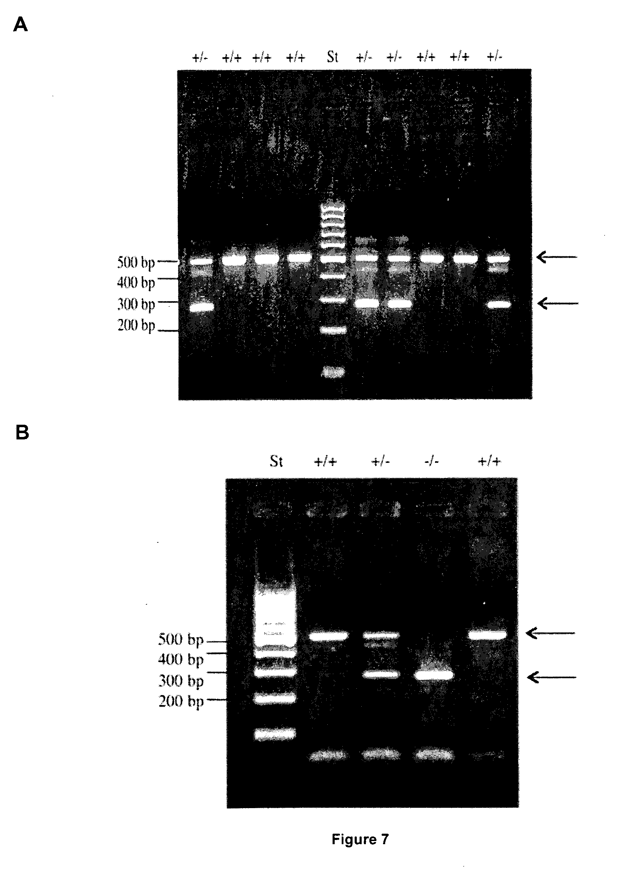

The positive ES-cell clones were injected into blastocysts of C57BL/6 mice, resulting in highly chimeric males, which were crossed with C57BL/6 female mice. The crossing of the chimeras of the ES-cell clone VI.1.1 with C57BL/6 females successfully generated heterozygous offspring carrying the introduced mutation in the CD22 gene. The heterozygous mice were further crossed, resulting in homozygous mice. For further analysis, the mice were screened by PCR. Heterozygous mice of the F1 generation showed two bands after PCR amplification - one at 500 basepairs for the wildtype allele and one at 300 basepairs for the knock-in allele - as shown in figure 7A. Mice of the F2 generation were also tested by PCR. All expected 3 genotypes were detected (500bp for wild type, 300 bp and 500 bp for heterozygous and 300 bp for homozygous knock-in mice)- as shown in figure 7B.

### Example 4: Analysis of the human CD22 expression on heterozygous and homozygous mice

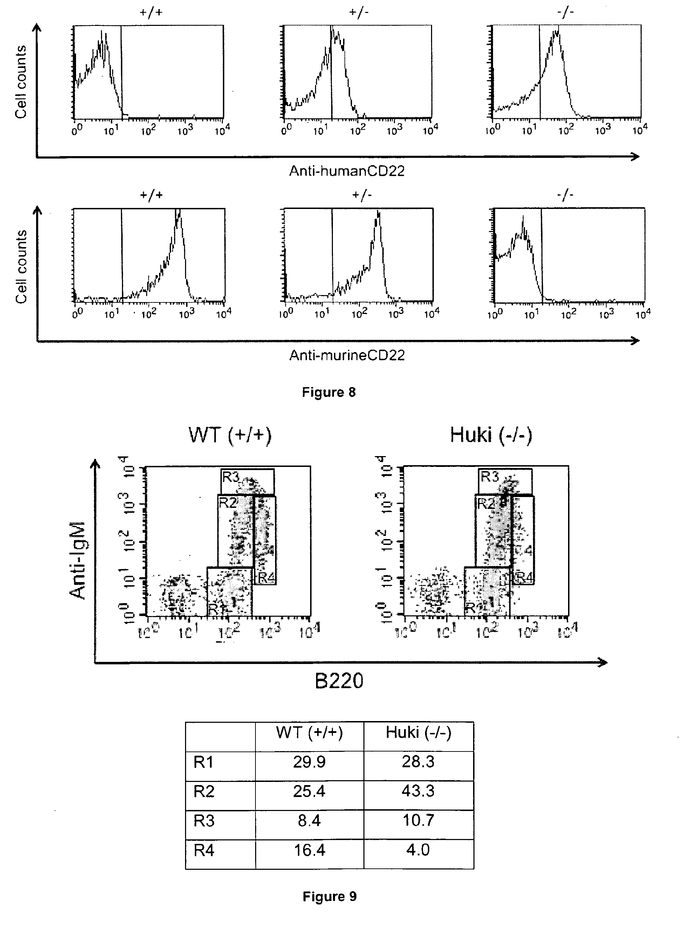

The heterozygous and homozygous knock-in mice were analyzed by flow cytometry. Erythrocytes were depleted from peripheral blood cell samples and cells were then stained with antibodies against human or murine CD22, together with the B cell marker B220. As shown in figure 8, homozygous knock-in mice express human CD22 but no murine CD22 on the surface of B220 positive cells.

### Example 5: Analysis of bone marrow development in Huki CD22-transgenic mice

Bone marrow cells of wild type and homozygous knock-in mice were analyzed by flow cytometry. The cells were taken from both femurs, the erythrocytes were depleted and the cells were stained with antibodies against IgM, together with the B cell marker B220. As shown in figure 9, the Huki CD22-transgenic mice have a normal B-cell development, showing all expected cell populations with only some minor changes in the proportions between the cell types.

### Example 6: Analysis of the expression of human CD22 on spleen and lymph node cells

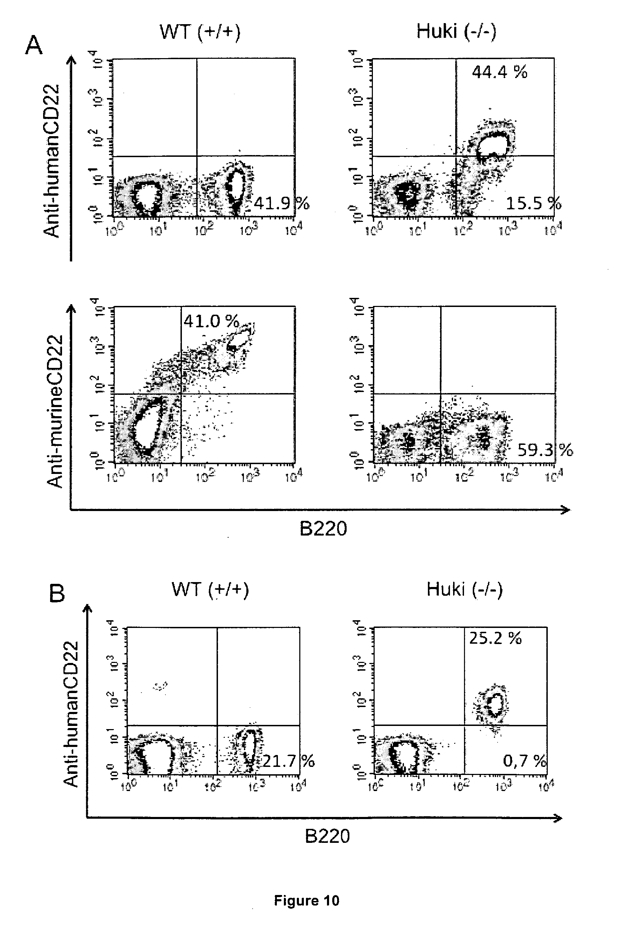

The spleen and lymph node cells of wild type and homozygous knock-in mice were also analyzed by flow cytometry. Single cell suspensions from spleen (after depletion of erythrocytes) and lymph nodes were stained with antibodies against murine or human CD22, together with the B cell marker B220. As shown in figure 10, the Huki CD22-transgenic mice express human CD22 on spleen and lymph node B cells, but no murine CD22. Some immature B cells (15.5 %) in the spleen of HuKi CD22-transgenic mice express low levels of human CD22.

These above results show that Huki CD22-transgenic mice possess normal B cells, similar to wild type mice. Both the populations of precursor B cells in the bone marrow, as well as mature B cells in spleen and lymph node develop in normal numbers. Consequently, human CD22 can replace murine CD22 without the occurrence of any defects in B cell maturation.

### References

Bolton, W. K., Chen, L., Hellmark, T., Fox, J., Wieslander, J. (2005). Molecular mapping of the goodpasture's epitope for Glomerulonephritis. Trans Am Clin Climatol Assoc 116, 229-36
Crocker, P.R., Paulson, J.C., and Varki, A. (2007). Siglecs and their roles in the immune system. Nat Rev Immunol 7, 255-266.
Crocker, P.R., and Redelinghuys, P. (2008). Siglecs as positive and negative regulators of the immune system. Biochem Soc Trans 36, 1467-1471.
Dorner, T., Jacobi, A.M., and Lipsky, P.E. (2009). B cells in autoimmunity. Arthritis Res Ther 11, 247.
Flavell, D.J., Noss, A., Pulford, K.A., Ling, N., and Flavell, S.U. (1997). Systemic therapy with 3BIT, a triple combination cocktail of anti-CD19, -CD22, and -CD38-saporin immunotoxins, is curative of human B-cell lymphoma in severe combined immunodeficient mice. Cancer Res 57, 4824-4829.
Ghetie, M.A., Richardson, J., Tucker, T., Jones, D., Uhr, J.W., and Vitetta, E.S. (1991). Antitumor activity of Fab' and IgG-anti-CD22 immunotoxins in disseminated human B lymphoma grown in mice with severe combined immunodeficiency disease: effect on tumor cells in extranodal sites. Cancer Res 51, 5876-5880.
Hurwitz, C.A., Loken, M.R., Graham, M.L., Karp, J.E., Borowitz, M.J., Pullen, D.J., and Civin, C.I. (1988). Asynchronous antigen expression in B lineage acute lymphoblastic leukemia. Blood 72, 299-307.
Kelm, S., Pelz, A., Schauer, R., Filbin, M., Tang, S., de Bellard, M.E., Schnaar, R.L., Mahoney, J.A., Hartnell, A., Bradfield, P., et.al. (1994). Sialoadhesin, myelin-associated glycoprotein and CD22 define a new family of sialic acid-dependent adhesion molecules of the immunoglobulin superfamily. Curr Biol 4(11), 965-72
Kreitman, R.J., Squires, D.R., Stetler-Stevenson, M., Noel, P., FitzGerald, D.J., Wilson, W.H., and Pastan, I. (2005). Phase I trial of recombinant immunotoxin RFB4(dsFv)-PE38 (BL22) in patients with B-cell malignancies. J Clin Oncol 23, 6719-6729.
Kulunian K, Wallace D, Petri M, Houssiau F, Pike M, Kilgallen B, Barry A, GordonC (2010). "Bilag-measured improvement in moderately and severely affected body systems in patients with systemic lupus erythematosus (sle) by epratuzumab: results from emblemTM, a phase iib study". EULAR Meeting 2010, Roma 2010, SAT0197
Kurtin, P.K. (2009). Indolent Lymphomas of mature B Lymphocytes. Hematol Oncol Clin North Am 23, 769-90
Leonard, J.P., and Goldenberg, D.M. (2007). Preclinical and clinical evaluation of epratuzumab (anti-CD22 IgG) in B-cell malignancies. Oncogene 26, 3704-3713.
Nitschke, L. (2009). CD22 and Siglec-G: B-cell inhibitory receptors with distinct functions. Immunol Rev 230, 128-143.
O'Gorman, S., Dagenais, N.A., Qian, M., and Marchuk, Y. (1997). Protamine-Cre recombinase transgenes efficiently recombine target sequences in the male germ line of mice, but not in embryonic stem cells. Proc Natl Acad Sci U S A 94, 14602-14607.
Pawlak-Byczkowska, E.J., Hansen, H.J., Dion, A.S., and Goldenberg, D.M. (1989). Two new monoclonal antibodies, EPB-1 and EPB-2, reactive with human lymphoma. Cancer Res 49, 4568-4577.
Pineles, D., Valente, A., Warren, B., Peterson, M. GE., Lehman, T.J.A., Moorthy, L.N. (2011). Worldwide incidence and prevalence of pediatric onset systemic lupus erythematosus. Lupus 0, 1-6.
Schirrmann, T., Krauss, J., Arndt, M.A., Rybak, S.M., and Dubel, S. (2009). Targeted therapeutic RNases (ImmunoRNases). Expert Opin Biol Ther 9, 79-95.
Szczepañ, T., van der Velden, V.H.J., van Dongen, J.J.M.(2003). Classification systems for acute and chronic leukemias. Best Pract Res Clin Haematol 16, 561-82
Townsend, M.J., Monroe, J.G., Chan, A.C. (2010). B-cell targeted therapies in human autoimmune diseases: an updated perspective. Immunol Rev 237, 264-83.
Walker, J.A., and Smith, K.G. (2008). CD22: an inhibitory enigma. Immunology 123, 314-325.
Wilmut, I., Hooper, M.L., Simons, J.P. (1991). Genetic manipulation of mammals and its appication in reproductive biology. J. Reprod. Fert 92 245-279.
Wilson, G.L., Najfeld, V., Kozlow, E., Menniger J., Ward, D., Kehrl, J.H. (1993). Genomic structure and chromosomal mapping of the human CD22 gene. J Immunol150, 5013-24.

## Claims

1. A genetically modified rodent comprising a nucleic acid sequence encoding human CD22, or (a) fragment(s) thereof, in its genome.

2. The genetically modified rodent of claim 1, wherein the human CD22, or fragment(s) thereof, is expressed on the surface of B lymphocytes.

3. The genetically modified rodent of claim 1 or 2, wherein the expression of human CD22, or fragment(s) thereof, is under the control of the endogenous rodent CD22 promoter.

4. The genetically modified rodent of any one of claims 1 to 3, wherein the expression of the endogenous nucleic acid sequence encoding rodent CD22 is disrupted.

5. The genetically modified rodent of any one of claims 1 to 4, wherein the rodent is a mouse.

6. The genetically modified rodent of claim 5, wherein the nucleic acid sequence encoding human CD22 is a cDNA, or (a) fragment(s) thereof, and is integrated into an exon 3' of the signal peptide sequence of the endogenous murine CD22 gene.

7. A method of identifying an agent capable of treating diseases associated with dysregulated B lymphocytes, the method comprising:
(a) administering an agent to the genetically modified rodent of any one of claims 1 to 6; and
(b) determining the amount and/or activity of B lymphocytes expressing human CD22, or (a) fragment(s) thereof, in the rodent after administration of the agent in step (a);
wherein a decrease in the amount of B lymphocytes determined in step (b) as compared to the amount of B lymphocytes prior to the administration of the agent in step (a) is indicative of an agent capable of treating diseases associated with dysregulated B lymphocytes.

8. The method of claim 7, wherein the diseases associated with dysregulated B lymphocytes are selected from the group consisting of autoimmune diseases, cancer and common variable immune deficiency (CVID).

9. The method of claim 8, wherein the autoimmune disease is selected from the group consisting of systemic lupus erythematosus (SLE), Rheumatoid arthritis, Goodpasture Syndrome and Multiple Sclerosis.

10. The method of claim 8, wherein the cancer is selected from the group consisting of B-cell lymphomas and B-cell leukemias.

11. A method of identifying an agent capable of treating autoimmune diseases associated with dysregulated B lymphocytes, the method comprising:
(a) administering an agent to a rodent animal model obtained from mating the genetically modified rodent of any one of claims 1 to 6 with a disease model; and
(b) determining the amount of disease-specific autoantibodies in the rodent after administration of the agent in step (a);
wherein a decrease in the amount of disease-specific autoantibodies determined in step (b) as compared to the amount of disease-specific autoantibodies prior to the administration of the agent in step (a) is indicative of an agent capable of treating autoimmune diseases associated with dysregulated B lymphocytes.

12. A method of testing the safety of an agent for anti-human CD22 therapy, the method comprising:
(a) administering the agent to the genetically modified rodent of any one of claims 1 to 6; and
(b) analysing said rodent for short or long term adverse effects.


SEQUENCE LISTING

<110> Friedrich-Alexander-Universität Erlangen-Nürnberg

<120> A genetically modified rodent expressing human CD22

<130> T2105 EP

<160> 13

<170> PatentIn version 3.5

<210> 1
<211> 3286
<212> DNA
<213> Homo sapiens

<400> 1
acttctcctt ttgctctcag atgctgccag ggtccctgaa gagggaagac acgcggaaac 60

aggcttgcac ccagacacga caccatgcat ctcctcggcc cctggctcct gctcctggtt 120

ctagaatact tggctttctc tgactcaagt aaatgggttt ttgagcaccc tgaaaccctc 180

tacgcctggg agggggcctg cgtctggatc ccctgcacct acagagccct agatggtgac 240

ctggaaagct tcatcctgtt ccacaatcct gagtataaca agaacacctc gaagtttgat 300

gggacaagac tctatgaaag cacaaaggat gggaaggttc cttctgagca gaaaagggtg 360

caattcctgg gagacaagaa taagaactgc acactgagta tccacccggt gcacctcaat 420

gacagtggtc agctggggct gaggatggag tccaagactg agaaatggat ggaacgaata 480

cacctcaatg tctctgaaag gccttttcca cctcatatcc agctccctcc agaaattcaa 540

gagtcccagg aagtcactct gacctgcttg ctgaatttct cctgctatgg gtatccgatc 600

caattgcagt ggctcctaga gggggttcca atgaggcagg ctgctgtcac ctcgacctcc 660

ttgaccatca agtctgtctt cacccggagc gagctcaagt tctccccaca gtggagtcac 720

catgggaaga ttgtgacctg ccagcttcag gatgcagatg ggaagttcct ctccaatgac 780

acggtgcagc tgaacgtgaa gcacaccccg aagttggaga tcaaggtcac tcccagtgat 840

gccatagtga gggaggggga ctctgtgacc atgacctgcg aggtcagcag cagcaacccg 900

gagtacacga cggtatcctg gctcaaggat gggacctcgc tgaagaagca gaatacattc 960

acgctaaacc tgcgcgaagt gaccaaggac cagagtggga agtactgctg tcaggtctcc 1020

aatgacgtgg gcccgggaag gtcggaagaa gtgttcctgc aagtgcagta tgccccggaa 1080

ccttccacgg ttcagatcct ccactcaccg gctgtggagg gaagtcaagt cgagtttctt 1140

tgcatgtcac tggccaatcc tcttccaaca aattacacgt ggtaccacaa tgggaaagaa 1200

atgcagggaa ggacagagga gaaagtccac atcccaaaga tcctcccctg gcacgctggg 1260

acttattcct gtgtggcaga aaacattctt ggtactggac agaggggccc gggagctgag 1320

ctggatgtcc agtatcctcc caagaaggtg accacagtga ttcaaaaccc catgccgatt 1380

cgagaaggag acacagtgac cctttcctgt aactacaatt ccagtaaccc cagtgttacc 1440

cggtatgaat ggaaacccca tggcgcctgg gaggagccat cgcttggggt gctgaagatc 1500

caaaacgttg gctgggacaa cacaaccatc gcctgcgcag cttgtaatag ttggtgctcg 1560
tgggcctccc ctgtcgccct gaatgtccag tatgcccccc gagacgtgag ggtccggaaa 1620
atcaagcccc tttccgagat tcactctgga aactcggtca gcctccaatg tgacttctca 1680
agcagccacc ccaaagaagt ccagttcttc tgggagaaaa atggcaggct tctggggaaa 1740
gaaagccagc tgaattttga ctccatctcc ccagaagatg ctgggagtta cagctgctgg 1800
gtgaacaact ccataggaca gacagcgtcc aaggcctgga cacttgaagt gctgtatgca 1860
cccaggaggc tgcgtgtgtc catgagcccg ggggaccaag tgatggaggg gaagagtgca 1920
accctgacct gtgagagcga cgccaaccct cccgtctccc actacacctg gtttgactgg 1980
aataaccaaa gcctccccta ccacagccag aagctgagat tggagccggt gaaggtccag 2040
cactcgggtg cctactggtg ccaggggacc aacagtgtgg gcaagggccg ttcgcctctc 2100
agcaccctca ccgtctacta tagcccggag accatcggca ggcgagtggc tgtgggactc 2160
gggtcctgcc tcgccatcct catcctggca atctgtgggc tcaagctcca gcgacgttgg 2220
aagaggacac agagccagca ggggcttcag gagaattcca gcggccagag cttctttgtg 2280
aggaataaaa aggttagaag ggcccccctc tctgaaggcc cccactccct gggatgctac 2340
aatccaatga tggaagatgg cattagctac accaccctgc gctttcccga gatgaacata 2400
ccacgaactg gagatgcaga gtcctcagag atgcagagac ctcccccgga ctgcgatgac 2460
acggtcactt attcagcatt gcacaagcgc caagtgggcg actatgagaa cgtcattcca 2520
gattttccag aagatgaggg gattcattac tcagagctga tccagtttgg ggtcggggag 2580
cggcctcagg cacaagaaaa tgtggactat gtgatcctca aacattgaca ctggatgggc 2640
tgcagcagag gcactggggg cagcgggggc cagggaagtc cccgagtttc cccagacacc 2700
gccacatggc ttcctcctgc gcgcatgtgc gcacacacac acacacacgc acacacacac 2760
acacacactc actgcggaga accttgtgcc tggctcagag ccagtctttt tggtgagggt 2820
aaccccaaac ctccaaaact cctgcccctg ttctcttcca ctctccttgc tacccagaaa 2880
tccatctaaa tacctgccct gacatgcaca cctccccctg cccccaccac ggccactggc 2940
catctccacc cccagctgct tgtgtccctc ctgggatctg ctcgtcatca tttttccttc 3000
ccttctccat ctctctggcc ctctacccct gatctgacat ccccactcac gaatattatg 3060
cccagtttct gcctctgagg gaaagcccag aaaaggacag aaacgaagta gaaaggggcc 3120
cagtcctggc ctggcttctc ctttggaagt gaggcattgc acagggagac gtacgtatca 3180
gcggcccctt gactctgggg actccgggtt tgagatggac acactggtgt ggattaacct 3240
gccagggaga cagagctcac aataaaaatg gctcagatgc cacttc 3286

<210> 2
<211> 54
<212> DNA
<213> Mus musculus

<400> 2
atgcgcgtcc attacctgtg gctcctcttg atcctaggac acgtggcttc ggct 54

<210> 3
<211> 14941
<212> DNA
<213> Mus musculus

<400> 3
acgcttataa ggacatttaa gtggggctgg cttacaggtt cagaggttca gtccattatc 60

atcaaggcag gaggatggca gcatccaggt tggcatggtg caggaggagc tgagagttct 120

acatcttcat gtgctaggag aaggctgcta ggagaaaact ggctaaagcc catgcccaca 180

atgacatacc tactccaaca aggccacgcc tcctaatatt accactccct aggccaagca 240

tattcaaacc atcacagctc gcatgcatga aaccctgagt tcaataccaa cactgtgaga 300

caatggtgcc tctagggact gatttgataa tgagattacc ctgagtcttt tcccctccct 360

ctaggcacca agaagccatc tccatctcca tgtttccagt gtgatcaatc accaatacaa 420

aggagttcat gtgacagctg cgccactttt aatatgaagc acttattgaa ttatatatgt 480

ccattccgtt ctgtctgctc agcgtccagg acccccgagg gaaggcacca tctccacaga 540

aggtccaaca tctttgtaga agaaaagcca actggggaca gtgcctcttg cttccttact 600

tctgtccttc ctggggattt ctccccaggg ccagaaagtg ggtagtaaga ttagtgaacg 660

gggtagagta tcaaggggtc agagaagaag tgttagtgac gattacaaca ggaacacatc 720

cagctgcagt gaggcacagg atggagcaga tggtggtggg tgcctggggt tggagaagca 780

gacgtggcac aatgcgtgag ggaggatcac cagccagaga aggccggtca ggtctggcca 840

gacacaggtt gttttttgtt tggttggttg gttggttttt catttttcca atacagggtt 900

tccttgaaaa accctggctg tcctggaacc cactctgtag agcaggctgg cctcgaactc 960

agaaatccgc ccgcctctgc ctcctgagtg ctggcgttaa aggcgtgcgc cacatgcacg 1020

cctgagtccg tgcccaggga gctgccggtg ccttagcatc tgagctgcag cagctgaagg 1080

agttgtcttc tttaggctca agaacctgaa acagcagctg ctacccacct cagtgcttga 1140

gggtcacata gtctacgtct tcctttgcct ggggccgctt accagcccca aactgaacca 1200

gctctgagta atggatgctc tcatcctccg ggcagctcgg attcacattc tcataatccc 1260

cctgcaaagc acatgaaggt gaggcaggcc ggggtgacca gccttcctcc attccctctc 1320

aagccttctc ccactgggtc cccaatgtcc aaaatgtggg gtctgtggga ggtgactata 1380

ttgatgggtt aatagataat caaggcagag actgttttag ggagttgagt tctctatggc 1440

atatgcccca gctgtgaaat aagcttcttt ccattataaa ctaccgggtc ttgcatattc 1500

aattatagca atagaaaata gacttaagtc tgccaccatt cccaacttca ctgtctgtgt 1560

gtcccatgac cagctctcag ttgagacccc agccttccat agctctgccc tcttaccata 1620

ggccgcttct gtatcaccga gtaagtgacc gagtcgctgt tgtttggagg aggagcctgt 1680

gttgctgggg tccctgcatc tctgagagaa aggagatggc tacacatgag ctttcccagc 1740

agcgcttcga ccccagcccc ttaggggagg acttctctgt gcacagcacc agagcagcag 1800

ctaatgaaaa tggctgtgtg aatgtatcac aacactgtca acctgccgtg cagcctggct 1860
ctggacacca gcttctgaac taaagacaca cgtcccttct gaggcctgtt accttgtcat 1920
gcagcaagaa taagggaatg agtgtacgtg tatggtgaag ccttgtacag gtccggcaca 1980
aagagggcag actaggacac actgttttcc tcccacaccc cttgggaatt acagctacca 2040
aactccagta ccctccttac ccagtgttgt gcgtgtcact ctctggaaag cgcaagatgg 2100
cataactaac ggtgtcatcc attgccggat tgtagcatcc ctgggattgg gggccttctg 2160
agagaggggt cctcctagcc tggaagatgc aaagtggaag ctatggctct cttctctgac 2220
cccatgcctt gcccctggcc tttgacccca acaattccct ggaggctttt tctcagtgct 2280
tctccagcac tgagactgca gatgcccgtg tgccctcaag cccttttctc atctgttgtc 2340
tccaagggct accctggatc tcatcttcct ctcgtcttac ctttttgttc ctcacaaaaa 2400
agctctggcc actggaattt tcctgaagcc cctgctggct gcggttttgc ttccatctaa 2460
ggatcaaggg gtttgtgagg gcaggtgggg atgatcctgg caggctgcgg gctgtgggga 2520
ggggacagga agaggagggg cagcagacag acaggctcca ctttttctgg attttcatcc 2580
cccagatggc caggatgcag atagtcaggc agaagcctag tcccaaggcg acccgcttgc 2640
cgatggtctc tggactgtct ggtgaagaaa ggtaaacagg cccactctgc cttgtgaagg 2700
cagagagaga gcattctgtc cttatccttt cacgaagcct ggtccttctt gcctgcagat 2760
ctcctcctca tatgggagat ggatagagaa gaggatggag aagaagtccc tgtctttcca 2820
cccctgatag cagagaagag acaaatcttt gcctctagca agagctactc catagtcagc 2880
tctcacagaa agcctgtgac ccttgagttg gttgctgccc tgcaaccctg agaacagttt 2940
gatcagacag tcaaaactta aagtgcaccc agacagactc tgctccaaca caagcctggt 3000
ccctggcttg tccttgcagg tgtgtccaca gcaagggttt cagttcgtct ctgccttgaa 3060
gagggtgcta gggagatggg atgggctaca gtcatttgaa agaggccagc acaaactggg 3120
catggttact cctatctgta atccccacac tcaggaggct gagcctggag ttcaaggcca 3180
acctgggctg catagcaaga ctcttatctc aaacaaaaaa gtgtgggtgg gttttgtagg 3240
aaagggagat tttttttaaa agatttattt attatatgta agtacactga agctgtcttc 3300
agacacacca gaagaaggca tcagatccca tacagatggt tgtgagcccc catgtggttg 3360
ctgggaattg aactcagaac ctctggaaga gcagtcaatg ctcttacctg ctgagtcatc 3420
tctccagccc aggagatgag gttcttttgt tttgtttttc tacatagccc aggctggcac 3480
tggccttcac atggcatcgc cactccaacc tctcaaggcc ctgttcagct tcctttttgt 3540
gcttgaaaag attttttaaa aaaactatat gctagtatta tcttgtttaa aacactaaaa 3600
tatcaaaaga taagaaataa ggatcttagc tgggtggtgg tggccaacac ctttagtcac 3660
agaacttggg aggcagaggc agaggcagag gcagaggcag aggcagaggc agaggcagag 3720
gcagaggcag aggcagaggc agaggcagag gcagaggcag aggcagaggc agaggcaggt 3780
agatctctgt gagttcatgg ccagcctggt ctatagagca agttctggga cagctatgac 3840

ttcacaggga aacccttcct tgaaaagcaa aaacaaaaca aaacaaaaca aaacaaaaat 3900
taaaaaaaaa ataaaagaac taagtgtctt atccatcaag ggcgagattg gcatgtagaa 3960
gaaaggagga aggcaggccg gaagagcagg ggactttcca ctcaggtcac agggtatgtc 4020
tgagctagac acaggagaag gaagagggaa gggactgcag ctcccaggac aggacaggga 4080
ccaagggaag acagcaaggg gaacttacag tagacagtga gggtgctggg tggtgactct 4140
cctgtgccta tcccattggt ccctttgcag cggtaggaac ccgtgtgttg gacctccagg 4200
ggttccagtc tcagtttctg gcctgaggag tggaggtctt ggccactgga gtcaaaccag 4260
gtgtactgtg agatgggcgg attggcatca ctctcacagg acaaggtggc cttcttcccc 4320
tccatcacat ggtccccagg gctgatggac acacgcagcc tccgaggagc atctgtaaag 4380
gagaccccga gtcaggccgg ggcagcgggt cacctcatca tcttctcatt taggccccag 4440
ctccagccac tcacacagca cttggaggtt ccaggcctgt gacaaggtct ctccgatgga 4500
gttgttgacc atgcagttat aatttccaga atcttctggg gagacggagc cgaagctcag 4560
gtacctccct tcctgcacga gactcccatt cttcttccag aagaagcgga cctctgccgg 4620
gttgctctct gcgaagtcgc attggaggag gacacgctgc ccagcgcgga tctctgatgc 4680
ggggcttacc ttcagtacct tcacgtctct gggggcgtct gcagggccac agcctcaggt 4740
gatgttttgg ttttcttccc ttcttgatgt ccccaaagtt tcctgtcccc ttgattagga 4800
caccctgtct ccttggcgct gagccctgtg cgtcctccct caaccctctc ttttggcctg 4860
tggcccgagt ccccatcccc tcccctcact ctcccaccca cagatctcaa tgtgacctcc 4920
tcctgggcac cccatatcac tcacagtgga cattcaggat gacagggagg gcccacgaac 4980
acttgtggtt gcaggcagca cagctgacag gcatggaatc ccatgtcact ttctgaatcc 5040
tcagcactcc gggtttgagc acactcccag aaccttgagg gttccatctg taggaggtga 5100
cgtctggatt gctggagttg tacctacaga ccagggtcac actgtctcct tccaggattg 5160
gtgtgaagct ctgaatcact gtggtcaccg ccttgggagc atcttagggt agaatggaga 5220
ggcaagcaaa gatggagagg caagcaaaga atgatttctt ttctttccct ttgcaagcac 5280
cagacaccac acgtgaggtc ttacatgggc taggcaagag ttctaccact gaatgaacta 5340
tatcactagc cctaacctat ttctctttct ttctttcttt ctttctttct ttctttcttt 5400
ctttctttct ttctttcttt ctttctttct ttctctctct ctctctctct ctctctctct 5460
ctctctctct ttctttcttt ctttcttttt ttctttttct tttttttttt ccaagatagg 5520
gtttctctgt gtagccctgg ctgtcctgga actcactttg tagaccaggc tggcctcgaa 5580
ctcagaaatc cacctgcctc tgcctcccga gtgctgggat taaaggtgtg tgccaccaca 5640
cccggctccc tatttttctt ttttaacctt ttatttttga gtcacagtct tcttaatttg 5700
cacaggtcag ccttgaactt gaatagcccc tgaataagcg aaaaggccga cttgcaccac 5760
cctgggccag ttgcaaggct ggttttcaca gaaccttttc acattgtccc tgaatgcatc 5820
ctctcctgtc ccctctcctg tcccctcttg cacctgtgaa gcagagccca tgctgtttaa 5880

agctgtgtga tggtggcctg tagaccccta tcaagttggg cacctagcct tctagaatct 5940
tcccaaagga tatccttaga gcagccagtg gtgtgatact actgctgttc agatgttcag 6000
agtatgggta gccctgtctg tatccaagcc actaggtacc caacaaacac tatcctggaa 6060
gcatctccaa ggcaagctgt gcagtgccga ggaatgccaa acagtggggc agaatggtga 6120
gatggtccct ctggctctca gatctatcag gacttcttca gggcctcacc cgttgcctac 6180
gaatgttaga tctccatgcc tcacatcgcc caggcaggcc tcaaacttgc tatagagcca 6240
aggtgacctt gaatttatga tcctcctgcc ttgtgtctta agtgctgaga tttcgggtat 6300
gtgccactac atttgtttct tttatgcggg gctggatgaa tccagagctg tatgtgtgct 6360
aagtattcta ccaagggagc caaaccccca acttctctat aggggatttt aatcaggggc 6420
tctaccactg agccacggcc ccagcccctc actgggggat tctgggcagg ggctctacca 6480
ctgagccacg gccccagccc ctcactgggg gattctagac aggggctcta ccactgagcc 6540
acgcccccag cccctcactg ggggattcta ggcaggggct ctaccactga gccatgcccc 6600
cagcccctca ctggggggat tctaggcagg ggctctacca ctgagccacg cccctagctc 6660
ctcactgggg gattctaggc aggggctcta ccgctgagcc atgcccccag cccctcactg 6720
ggagattcta ggcaggggct ctatcactga gccatgcccc cagatccctc actgggggat 6780
tccagactag tgtttggttg ctgagtacct tactggtata ttgtaggttg tattctatta 6840
ttgagctaca tctctggtcc tctttctact tttgagccag tcttactaag ttgcctaggc 6900
tgaacttgaa tttactctgc tgcccaggca ggcctcacgt ttgtgatttt tcagcttccc 6960
cagtagctgg gatgacagat ctgggccacc aggctggtga gattcattca ttcattcatt 7020
cattcattca ttcattcaac aaacatctac taagtaagtg ccataagctg aggctcttgg 7080
tcactgtcta gaatgcagct gtgaagctcc aggtgcctgg tggcttgtct tccaaactcc 7140
ctcccacccc ctcatcctac acccagtaca ccccttcacc tccacaccca tcactcccta 7200
tacctccaca ccccaacaca ctcaacaccc actacacccc ctcactccca caacccctca 7260
cccctacacc ctccctattt gccttcctta tttccctctt tcctccaaaa cccagaagtc 7320
agttggccgc tcacaatgga catccagctt agcttcctgg tctatctttc catgacccag 7380
acggttctct gccaagcaag agtaattccc agcatgccac ggggagactt tagggatgcg 7440
gagcttctct tgggtgtctc caggtatagg tttcctgttg tgataccagg tgtagtttgt 7500
tgcacttgga ctggccagtg actcacaaat cagctctact gactgtccct cttcagcggg 7560
ggaagggtag atgtgaaccc tggagggttc tggagcatct gaaagaatgt atggccacag 7620
gacaggtgag cagagcctgc taatgtgggc aggcagccac ctgaacctct ggtatcatcc 7680
cttgaggatc ctgctaactc cgcacctccc tcaccagctt caaggaaact cacagtgcac 7740
cgtgagttcc acttcttccg actctcctgg gcctatgtcg ttggaagcct ggcaccggta 7800
tttccctctc atgtccttgg tcactgaatg cagaattagc ttggacatct gttgttcctg 7860
ttccagttcc tgatcctcta gggggcgccc atccttgaac caagacaccg ccacggtcct 7920

gagtttcggg ttgctgctgt taacccggca tgtcatggtc acagaattgt tcttttccac 7980
ctctgtggga ttgaccttga tctccagctt cggggtatct gcgggaaacg agaagcagtt 8040
gcggagagca cagggaatgc tcggtggctg cactgttatt ccgacagcag tagcacgcac 8100
atctgtggtt cctatgtcca cccaggtgac tgccccagct cctcacttgt ctgcctactc 8160
tcatttctcc tgtcccctac ccttgttgtc tctgccggca ccccgagtat ccctagcatc 8220
gtcaaagact ggcactgaag ctgtctatcc actgggcttg tacagcccag gggaccagcc 8280
ctctctactt ctttccctgc ttagctgaaa aggctcaggc ttgacccaca ctcctctccg 8340
gtcctcggcc actgccgtgg gtacacgatg ctccccctca ggacctttgt gcttatttct 8400
ttctctccat gggaacttct ttgcctgttc tctatcatgt cagctgaggt ctgcaccttc 8460
ctcacatgag tctctatcct tctaatcacc cgcctccttt tgaccggttt ttgaggtatt 8520
tgtgcgcgcc cgcgagtgcg caggtgacta tggtcagagg acagccatgg gtgttgtgct 8580
tcagaaacca tccatcttgt ttttacattt tttgagacca ggtctctcgc tgatctggag 8640
ctctattaga ccagcccaag gatccacccg gatctgcctc cccacggctg agacaacaag 8700
caaaggccac catacccaac ttgttttgtt ttgttttgtt ggctttttgt tcttccagac 8760
gggggtttct ggaactcaca ctgtagacca ggctggcctc gaactcagat ccacctgcct 8820
ctgcctccca agtgctagga ttacaccaca cgctgcttac attcaacgct taaaatgtga 8880
gctctgagcc gggtgtggtg gcgcacgcct tttatcccag cactcaggag gcagaggcag 8940
gcagatttct gagttcgagg ccagcctggt ctacagagtg agttccagga cagccagggc 9000
tatacagaga aaccctgtct tgaaaaacca aaaaaaaaaa aaaaaaaaag aatgtgagct 9060
ctggggttca aactcaagtc ctcattcttg taaggcaaat acatttaaaa aaaaagtttg 9120
ttttcttttc tttgtgtgta tctgtgtcag agggacggcc tcagatccca tggagagaca 9180
gctcttggtg gttgtcagct gcctggtgtg gatgctatga attgaattca ggtcctctgc 9240
aagagcagta agtgctctaa accattgaac tatctttcct aagtacttaa aaaaatatat 9300
tatggggctg gagagatggc tcggcagtta agagcactgg ttgctcttcc agaggactag 9360
ggtttccagc atctacaggg aggctcacga ctgtctgtaa ctccagttcc agggcatcca 9420
acaccctctt ctgggctcca caaacaccat tcttgtgatc cgtgcactgg catacattca 9480
ggcagaacac tcacacacat acaaataaaa aattaaaagt cttttaaaat tgaataatca 9540
gtatatcaca tagtactaag ctttaaaaaa aataattcaa agggggttta ttagattggc 9600
tagacaaatt cagagttatc tacccagacc tctttttcta ttaatataca tatctcctcc 9660
tggcaattct gttctatgtt tcttatgtgt ctattccatt tcaactgtta atccacacag 9720
gcagaacctg tgtgctcagc ctaagctggc attcaagaca agactggcac ccaatcaatt 9780
cacaagcttg ggtgtttgtt ttaaggtagg gtctgattgg cttagaactc actatgtaga 9840
ccaggctaac ctcaaactca cagaactcca cctacttctg cctcatgcat gctgggttta 9900
aatgtatgta gcaccatgcc aggccccaca gtaaatattc attcagtggg tgaatgaatg 9960

ggtgaatgaa gcataaaata atgatgtgaa aacttacatg tatggagaac aatgaactct 10020
aaggagatga tcctcaaagc atgatgtggc ttcagaattg attgattgac tgactgatgg 10080
atggatggat tgattgatgc tggaggttag tcctgggtct cacaagtgct aagcaagcac 10140
cataccactg agctacatct ctagcccttg gtttttgaga ccacagtgtt ttactcccct 10200
gcccagtgcc cgattccagg cctgcattac cacacttaac tgggttgcct tattgttcag 10260
gccaaggcag aggctaaaat gaacctgtgt cttgagacac aggactaaca gaagagacag 10320
ttgtgccacc gcagaaacag gtgactgaca gctaaacctt cagcggacac aggctcttcc 10380
caccctgggt acaacgcaac aggatttagg aagaaggtga caggtagcat ccttcccacc 10440
cggagactca cacttaacat ccagacgcac agtgcgctct gagagcactt tggaggagtg 10500
ctggacttgg cacttcacac tctttccgtg gtccgtccac tttggttgga aggtgagctt 10560
gctctctgta tagacattct taatggagga ggtgacagaa gaggtgatgg aggtgacaga 10620
agaggtgatg gaggtgatct cagaatcttc taggaaccac ttcaagagga tgtcataccc 10680
aaagcaggag aaattcagtc cacaggtcag agtgacactt tgggattccc ggatttctga 10740
tggcatctgg atgtaaggtt ggaacggttt ctctaagggg aaagaagaga gaggcctgct 10800
tgctggagat gggacacaga ctggctgtcc acccctgact tcaggcctcc tctcccatgt 10860
gaccccagaa gaagcccttc aaggctagcc tttctttagt cagttcgact actctggaag 10920
gccttgggaa cacaagcctg ctgggaaatt gtgtttaaca ttgactttgt ttctcacttt 10980
ctccttccat tatgttccta atggcctcag gatcacccag ggtgatttga tttgatttga 11040
ttgattgatt gattgattga ttggtttaat tctccatgta tcattggtgg cacgtgtttg 11100
tttgtcagac tcagacttag agtcagccaa gcacaacatt cctttagtgg ggtgatcctg 11160
aggccctggg ctcaaatctc caccatgcca gaccatctga cagatgatgt gacaccaaga 11220
gacgcagcat ggacagctct agtgtggagt gatgatactg tctagggcag aggcccctgg 11280
ctgttgttag aggtcatgtc tgagccctgt gctctcctct agcctaaaag aatttggtca 11340
ttaacacact gagagatttg aaaatccaga cctctctctg ctttggaaag cttcagaaaa 11400
cctgagagcc tcgacaacgg attccagaac agacacaagc tgaggagaga aagcctgagg 11460
taggacaatt gctcatgctg cagttggctg tgtgtgtgtg agagagttat gtgagtgagt 11520
gagtgtgtgt gtgtgtgtgt gcatcaaagc ttcctatttg gtgagcaaat gaaaaggaga 11580
aataaaaagg aaagaacatg attgctccta ggtgtggtgg aagagaaatt atattgtaga 11640
taaaagggag aacatagcca gaggcaggca catctgggag agtccagagt ggatatgacc 11700
ccgagccagg tgaggaaggg tgggagaggg gaaccaggta cagcagccaa ggaaatgggc 11760
tagaggtaaa agagggtggg ctacccagat ggctggatta tatagggaag agcctggggg 11820
aggggcagcc cggctcttgg gctggggaga tgggctagag agtgggggcc aggagccagc 11880
cattctttgt aataggtagg gactgaggga tgctgggaga atctggtgcc aggccctctt 11940
taatatgtta aataggcacc ttggctgttt gctccagggt ttgagcaggt ccctctttgc 12000

tgccgctaga acagttggct gtctccccat gccttacccg agacattgag gtgaatgggc 12060
tccatccatc gttcagtccc tgcggtcatc ctcaacccca gattcccact gtcattggca 12120
cgtatcgggt ggattttcag ggtacaattg tctattctgt tccccagaaa tgttactctc 12180
ccttgcttag aaaggtacag ctcagactct gggtccttct cagtctttgt ggcgttgtac 12240
aggacagttc ctgtgaattt cttggtggcc ttgtcaaact catagttctg aaaaaggagg 12300
atgttgtcca gacgtgcctt gggtagtgga gttttgtact tgcaaggaat cctgatgcag 12360
gctccctccc aggcaaagag ggtttgggga tggtcaacgg tccaatcatt tgctgagctg 12420
taccgagccg aagccacgtg tcctaaaggc cacaactctg gtcagaagtt gttactgagg 12480
ggtgagaact tccactggaa gtaggagagg gagacactgg ggagacacac ggtggagcag 12540
gagcacagaa tctctgcggt tcccctccaa tgccccaggt ccatcccctc tctgcattat 12600
ccactgatat ctccagaccc agctaccagc ctgggcctca gcacttacct aggatcaaga 12660
ggagccacag gtaatggacg cgcatggtgt ctcgtctggg tgggacctaa agaaggaaga 12720
attagtctgt aaactggatg tgttagcatg tgactgtaat ctcagcattc aggaagttga 12780
gtaaggagaa ttgctatgag tctgaggcca gcgtgggcta cattctgaga cccggtctca 12840
acaagaacaa caaaaggaat ttgcctttga gccatcttgt tgctccctta tcttgtttgt 12900
tgtcccctga gttctggccc ctttcttcct gattttgtgt gtgtgtgtgt gtgtgtgtgt 12960
gtgtgtgtgt gtgtgtgtgt ggtgtgtcta gttatagtga tagagatgtg ggtagagccc 13020
ctgcctaaca agcacaggat ctagattcaa ttgtactagt actagcccaa aaagtaaaac 13080
taaactcata gtgcagtata tttggaggaa gttggaaagc cttagagcag cgagaaatct 13140
gagcgattgc ctcctgagcc ctgggtgctg atagggagca aggggcaggc atcccttttc 13200
taacaacagc cagtcgttag caggtgggaa tggggacctc atgttgtccg atcacccact 13260
gtctgaccat aaggaagaag ccagattgtc atgagaaatg tcttgcttta gaagcaatac 13320
ataggaccag ccatagtggt atatacctaa aaatcccaga attttggagc ctggatctat 13380
gaatacaagc ttgaagctag tctggcttac caagtcatta tgagtagtat taaataagta 13440
gtagcataca gctctagcct gacggatcaa tttaagggca actaatttac aactcttatc 13500
tctctctctc tctctctctc tctctctctc tctctctctc tctctgtctc tctctctctc 13560
tctctctctc tctctccaac aagcttggcc tccaccatcc tacactttat gtagagttaa 13620
atttaacaat ttaactctat tcatgtggct gtgatctctg tgtgagtccc cagggatctt 13680
gcaggatgtg ccaacagagt gtgtctccgg tgtccgagag cagagctgtg tcccttcgct 13740
tcccttccct tccacccacc caaggtttcc cataccttgc acagcatcct cacctccctc 13800
tctctcctgc tatttctctc ccaggccgca gggcacaggt agtaaggact tctgggtgac 13860
caggaacagg tgtagggaaa gccagttcag ttgctgagcg tgggctgggt tctgcctcca 13920
cggttggggg tggtgtggcc tgggtgtata taagaggttt gtgggctgag gctggagcag 13980
atcatctgag gaagagctta tcttagtttc ccaggtacag ggaatgcacc acatagagat 14040

gtcttcaaag tcacagccac ttgggaaaag cagagctcaa caggaggctt cactgcagga 14100

cttcttaagt tgtctaaaga gcttagacag gcagtggcca tgcttctttg gccacgagct 14160

ctattttaat cttttccaat gaaagcgttg cagggttgca agctctgttc tggaggacac 14220

actttaacac accggtgggg ttgctataac aagggctgtg gtgacaatca gcctctgggt 14280

gctggaccac cctgtttgtc cggcagcccc tccagccaca tccttagcag agcctggctg 14340

gagaactcaa gcttgagcag gaagcaggca gttccagagt gaagctctga accccaggaa 14400

tttacagcat gagaagcagg gacctgagcc tggggcccgg acgggtgaag aggtagagca 14460

gggtgcagca gcaacagtta ctaagtccag aagcttagaa cagggccaag aactgtgatc 14520

tgctcacctt ctgagagcct caaggttagc ttcctggtcc catcatttcc tccctggcac 14580

tcaaaagctt cttctacctc agaagaccca gaaagcctgg aaaggggaca ctgaacgaca 14640

gggatgggga gaacagggta gggatgaggg gttggggttg ggggtttagg cgttggaggg 14700

tgaggtttgt ctgtgcctat tgtaaagtag atgaaaaaac acagcaggcc ctcgaggcca 14760

gcacacagag gccactccat atccactcca tatttacttg tcgagaacaa gagaaaagat 14820

tgaaatggtt ttacctgttt cctcctgtgt tcgatcttca ggatccctag caggatctga 14880

gagtaagaaa actaagaaaa gtctcacacg cttggccacg cccccttcct ctctgggcct 14940

c 14941

<210> 4
<211> 66
<212> DNA
<213> Artificial sequence

<220>
<223> /note="Description of artificial sequence: primer polyA-Bsu15Ifw"

<400> 4
atatcgatat tcggacgtga ctgacatatg cgctctgtac accccctgaa cctgaaacat 60

aaaatg 66

<210> 5
<211> 27
<212> DNA
<213> Artificial sequence

<220>
<223> /note="Description of artificial sequence: primer polyA-BamHIrev"

<400> 5
atggatccac tcaaccctat ctcggtc 27

<210> 6
<211> 35
<212> DNA
<213> Artificial sequence

<220>
<223> /note="Description of artificial sequence: primer cDNA-PciIrev"

<400> 6

tctgtacatc aatgtttgag gatcacatag tccac 35

<210> 7
<211> 70
<212> DNA
<213> Artificial sequence

<220>
<223> /note="Description of artificial sequence: primer cDNA-Bsu15Ifw"

<400> 7
atatcgatat gctaagtagt gtattgtagg acatgtggct tcggctgact caagtaaatg 60

ggtttttgag 70

<210> 8
<211> 26
<212> DNA
<213> Artificial sequence

<220>
<223> /note="Description of artificial sequence: primer 22prom-LAfw"

<400> 8
ttatcgatga tgctgtatgg tactgg 26

<210> 9
<211> 21
<212> DNA
<213> Artificial sequence

<220>
<223> /note="Description of artificial sequence: primer 22prom-LArev"

<400> 9
gctttcgctt gctgatacat c 21

<210> 10
<211> 20
<212> DNA
<213> Artificial sequence

<220>
<223> /note="Description of artificial sequence: nested PCR primer for
the first PCR 1"

<400> 10
tgatccgtgc actggcatac 20

<210> 11
<211> 20
<212> DNA
<213> Artificial sequence

<220>
<223> /note="Description of artificial sequence: nested PCR primer for
the first PCR 2"

<400> 11
cggtatcgcc gctcccgatt 20

<210> 12

<211>  20
<212>  DNA
<213>  Artificial sequence

<220>
<223>  /note="Description of artificial sequence: nested PCR primer for
       the second PCR 1"

<400>  12
acctgtgtgc tcagcctaag                                                20


<210>  13
<211>  21
<212>  DNA
<213>  Artificial sequence

<220>
<223>  /note="Description of artificial sequence: nested PCR primer for
       the second PCR 2"

<400>  13
cgccttctat ctccttcttg a                                              21